(19) **Europäisches Patentamt / European Patent Office / Office européen des brevets**

(11) **EP 3 077 826 B1**

(12) # EUROPÄISCHE PATENTSCHRIFT

(45) Veröffentlichungstag und Bekanntmachung des Hinweises auf die Patenterteilung:
**13.05.2020  Patentblatt 2020/20**

(21) Anmeldenummer: **14809826.2**

(22) Anmeldetag: **05.12.2014**

(51) Int Cl.:
*G01N 33/68* *(2006.01)*

(86) Internationale Anmeldenummer:
**PCT/EP2014/076778**

(87) Internationale Veröffentlichungsnummer:
**WO 2015/082706 (11.06.2015 Gazette 2015/23)**

(54) **VERFAHREN UND MITTEL ZUR NICHTINVASIVEN DIAGNOSE VON TYP-II-DIABETES MELLITUS**

METHOD AND MEANS FOR THE NON-INVASIVE DIAGNOSIS OF TYPE II DIABETES MELLITUS

PROCÉDÉ ET DISPOSITIF DE DIAGNOSTIC NON INVASIF DE DIABÈTE SUCRÉ DE TYPE II

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2013  DE 102013224978**

(43) Veröffentlichungstag der Anmeldung:
**12.10.2016  Patentblatt 2016/41**

(73) Patentinhaber:
- **Universität Leipzig**
  **04109 Leipzig (DE)**
- **Ohio University**
  **Athens, OH 45701 (US)**

(72) Erfinder:
- **HOFFMANN, Ralf**
  **04463 Großpösna (DE)**
- **FROLOV, Andrej**
  **04159 Leipzig (DE)**
- **SPILLER, Sandro**
  **04299 Leipzig (DE)**
- **LI, Yichao**
  **Athens, OH 45701 (US)**
- **WELCH, Lonnie R.**
  **Athens, OH 45701 (US)**

(74) Vertreter: **Kailuweit & Uhlemann Patentanwälte Partnerschaft mbB**
**Bamberger Straße 49**
**01187 Dresden (DE)**

(56) Entgegenhaltungen:

EP-A2- 0 257 421      WO-A2-2005/114190
WO-A2-2007/117794    US-A1- 2010 158 916
US-A1- 2011 136 160

- PHILIPPE RONDEAU ET AL: "The glycation of albumin: Structural and functional impacts", BIOCHIMIE, MASSON, PARIS, FR, Bd. 93, Nr. 4, 7. Dezember 2010 (2010-12-07), Seiten 645-658, XP028172251, ISSN: 0300-9084, DOI: 10.1016/J.BIOCHI.2010.12.003 [gefunden am 2010-12-16]
- TAKAAKI GOTO ET AL: "Complete amino acid sequencing and immunoaffinity clean-up can facilitate screening of various chemical modifications on human serum albumin", ANALYTICAL AND BIOANALYTICAL CHEMISTRY, Bd. 405, Nr. 23, 12. Juli 2013 (2013-07-12), Seiten 7383-7395, XP055176048, ISSN: 1618-2642, DOI: 10.1007/s00216-013-7146-0
- KAZUTOMI YOSHIUCHI ET AL: "Glycated Albumin is a Better Indicator for Glucose Excursion than Glycated Hemoglobin in Type 1 and Type 2 Diabetes", ENDOCRINE JOURNAL, Bd. 55, Nr. 3, 1. Januar 2008 (2008-01-01) , Seiten 503-507, XP055175928, ISSN: 0918-8959, DOI: 10.1507/endocrj.K07E-089
- C. R. BORGES ET AL: "Building Multidimensional Biomarker Views of Type 2 Diabetes on the Basis of Protein Microheterogeneity", CLINICAL CHEMISTRY, Bd. 57, Nr. 5, 14. März 2011 (2011-03-14), Seiten 719-728, XP055176153, ISSN: 0009-9147, DOI: 10.1373/clinchem.2010.156976

SEQUENCE LISTING

<110> University Leipzig

<120> Verfahren und Mittel zur nichtinvasiven Diagnose von Typ-II-Diabetes mellitus

<130> 00401P0076DEWO

<160> 46

<170> PatentIn version 3.5

<210> 1
<211> 22
<212> PRT
<213> Homo sapiens

<220>
<221> MISC_FEATURE
<222> (2)..(2)
<223> Cysteine may be alkylated

<220>
<221> MISC_FEATURE
<222> (11)..(11)
<223> Cysteine may be alkylated

<220>
<221> MISC_FEATURE
<222> (13)..(13)
<223> glycated

<400> 1

```
Thr Cys Val Ala Asp Glu Ser Ala Glu Asn Cys Asp Lys Ser Leu His
1               5                   10                  15

Thr Leu Phe Gly Asp Lys
              20
```

<210> 2
<211> 17
<212> PRT
<213> Homo sapiens

<220>
<221> MISC_FEATURE
<222> (9)..(9)
<223> glycated

<220>
<221> MISC_FEATURE
<222> (11)..(11)
<223> Cysteine may be alkylated

<400> 2

```
Ser Leu His Thr Leu Phe Gly Asp Lys Leu Cys Thr Val Ala Thr Leu

     1               5                   10                  15

     Arg
```

<210> 3
<211> 17
<212> PRT
<213> Homo sapiens

<220>
<221> MISC_FEATURE
<222> (9)..(9)
<223> Cysteine may be alkylated

<220>
<221> MISC_FEATURE
<222> (10)..(10)
<223> Cysteine may be alkylated

<220>
<221> MISC_FEATURE
<222> (12)..(12)
<223> glycated

<400> 3

```
Glu Thr Tyr Gly Glu Met Ala Asp Cys Cys Ala Lys Gln Glu Pro Glu
1               5               10                  15

        Arg
```

<210> 4
<211> 17
<212> PRT
<213> Homo sapiens

<220>
<221> MISC_FEATURE
<222> (6)..(6)
<223> Methionine may be oxidized

<220>
<221> MISC_FEATURE
<222> (9)..(9)
<223> Cysteine may be alkylated

<220>
<221> MISC_FEATURE
<222> (10)..(10)
<223> Cysteine may be alkylated

<220>
<221> MISC_FEATURE
<222> (12)..(12)
<223> glycated

<400> 4

```
Glu Thr Tyr Gly Glu Met Ala Asp Cys Cys Ala Lys Gln Glu Pro Glu
1               5               10                  15


        Arg
```

<210> 5
<211> 19
<212> PRT
<213> Homo sapiens

<220>
<221> MISC_FEATURE
<222> (6)..(6)
<223> Cysteine may be alkylated

<220>
<221> MISC_FEATURE
<222> (7)..(7)
<223> Cysteine may be alkylated

<220>
<221> MISC_FEATURE
<222> (12)..(12)
<223> glycated

<220>
<221> MISC_FEATURE
<222> (15)..(15)
<223> Cysteine may be alkylated

<400> 5

```
    Ala Ala Phe Thr Glu Cys Cys Gln Ala Ala Asp Lys Ala Ala Cys Leu
    1               5                   10                  15

    Leu Pro Lys
```

<210> 6
<211> 16
<212> PRT
<213> Homo sapiens

<220>
<221> MISC_FEATURE
<222> (3)..(3)
<223> Cysteine may be alkylated

<220>
<221> MISC_FEATURE
<222> (7)..(7)
<223> glycated

<400> 6

```
    Ala Ala Cys Leu Leu Pro Lys Leu Asp Glu Leu Arg Asp Glu Gly Lys
    1               5                   10                  15
```

<210> 7
<211> 15
<212> PRT
<213> Homo sapiens

<220>
<221> MISC_FEATURE
<222> (7)..(7)
<223> glycated

<400> 7

```
    Ala Glu Phe Ala Glu Val Ser Lys Leu Val Thr Asp Leu Thr Lys
    1               5                   10                  15
```

<210> 8
<211> 17
<212> PRT
<213> Homo sapiens

<220>
<221> MISC_FEATURE
<222> (5)..(5)
<223> glycated

<220>
<221> MISC_FEATURE
<222> (8).. (8)
<223> Cysteine may be alkylated

<400> 8

```
Ala Asp Leu Ala Lys Tyr Ile Cys Glu Asn Gln Asp Ser Ile Ser Ser
1               5               10              15

Lys
```

<210> 9
<211> 21
<212> PRT
<213> Homo sapiens

<220>
<221> MISC_FEATURE
<222> (8).. (8)
<223> glycated

<220>
<221> MISC_FEATURE
<222> (9)..(9)
<223> Cysteine may be alkylated

<220>
<221> MISC_FEATURE
<222> (10)..(10)
<223> Cysteine may be alkylated

<220>
<221> MISC_FEATURE
<222> (18)..(18)
<223> Cysteine may be alkylated

<400> 9

```
Thr Tyr Glu Thr Thr Leu Glu Lys Cys Cys Ala Ala Ala Asp Pro His
1               5               10              15

Glu Cys Tyr Ala Lys
            20
```

<210> 10
<211> 17
<212> PRT
<213> Homo sapiens

<220>
<221> MISC_FEATURE
<222> (6)..(6)
<223> glycated

<400> 10

```
        Val Phe Asp Glu Phe Lys Pro Leu Val Glu Glu Pro Gln Asn Leu Ile
        1               5               10                  15

                Lys
```

<210> 11
<211> 15
<212> PRT
<213> Homo sapiens

<220>
<221> MISC_FEATURE
<222> (1)..(1)
<223> glycated

<400> 11

```
        Lys Val Pro Gln Val Ser Thr Pro Thr Leu Val Glu Val Ser Arg
        1               5               10                  15
```

<210> 12
<211> 10
<212> PRT
<213> Homo sapiens

<220>
<221> MISC_FEATURE
<222> (1)..(1)
<223> glycated

<400> 12

```
        Lys Gln Thr Ala Leu Val Glu Leu Val Lys
        1               5               10
```

<210> 13
<211> 16
<212> PRT
<213> Homo sapiens

<220>
<221> MISC_FEATURE
<222> (4)..(4)
<223> glycated

<400> 13

```
        Glu Gln Leu Lys Ala Val Met Asp Asp Phe Ala Ala Phe Val Glu Lys
        1               5               10                  15
```

<210> 14
<211> 12
<212> PRT
<213> Homo sapiens

<220>
<221> MISC_FEATURE

<222> (1)..(1)
<223> glycated

<400> 14

```
            Lys Leu Val Ala Ala Ser Gln Ala Ala Leu Gly Leu
            1               5                   10
```

<210> 15
<211> 24
<212> PRT
<213> Homo sapiens

<220>
<221> MISC_FEATURE
<222> (4)..(4)
<223> glycated

<400> 15

```
        Val Gln Trp Lys Val Asp Asn Ala Leu Gln Ser Gly Asn Ser Gln Glu
        1               5                   10                  15

        Ser Val Thr Glu Gln Asp Ser Lys
                        20
```

<210> 16
<211> 19
<212> PRT
<213> Homo sapiens

<220>
<221> MISC_FEATURE
<222> (14)..(14)
<223> glycated

<400> 16

```
        Asp Ser Thr Tyr Ser Leu Ser Ser Thr Leu Thr Leu Ser Lys Ala Asp
        1               5                   10                  15

        Tyr Glu Lys
```

<210> 17
<211> 21
<212> PRT
<213> Homo sapiens

<220>
<221> MISC_FEATURE
<222> (4)..(4)
<223> Cysteine may be alkylated

<220>
<221> MISC_FEATURE
<222> (17)..(17)
<223> glycated

<400> 17

```
Val Tyr Ala Cys Glu Val Thr His Gln Gly Leu Ser Ser Pro Val Thr
1               5                   10                  15

Lys Ser Phe Asn Arg
            20
```

<210> 18
<211> 18
<212> PRT
<213> Homo sapiens

<220>
<221> MISC_FEATURE
<222> (3)..(3)
<223> glycated

<400> 18

```
Gln Val Lys Asp Asn Glu Asn Val Val Asn Glu Tyr Ser Ser Glu Leu
1               5                   10                  15

Glu Lys
```

<210> 19
<211> 16
<212> PRT
<213> Homo sapiens

<220>
<221> MISC_FEATURE
<222> (3)..(3)
<223> glycated

<220>
<221> MISC_FEATURE
<222> (12)..(12)
<223> Methionine may be oxidized

<220>
<221> MISC_FEATURE
<222> (15)..(15)
<223> Cysteine may be alkylated

<400> 19

```
Ile Gln Lys Leu Glu Ser Asp Val Ser Ala Gln Met Glu Tyr Cys Arg
1               5                   10                  15
```

<210> 20
<211> 19
<212> PRT
<213> Homo sapiens

<220>

<221> MISC_FEATURE
<222> (1)..(1)
<223> glycated

<400> 20

```
Lys Trp Asp Pro Tyr Lys Gln Gly Phe Gly Asn Val Ala Thr Asn Thr
1               5               10              15

Asp Gly Lys
```

<210> 21
<211> 13
<212> PRT
<213> Homo sapiens

<220>
<221> MISC_FEATURE
<222> (2)..(2)
<223> glycated

<400> 21

```
Ser Lys Ala Ile Gly Tyr Leu Asn Thr Gly Tyr Gln Arg
1               5               10
```

<210> 22
<211> 16
<212> PRT
<213> Homo sapiens

<220>
<221> MISC_FEATURE
<222> (15)..(15)
<223> glycated

<400> 22

```
Ala Leu Leu Ala Tyr Ala Phe Ala Leu Ala Gly Asn Gln Asp Lys Arg
1               5               10              15
```

<210> 23
<211> 14
<212> PRT
<213> Homo sapiens

<220>
<221> MISC_FEATURE
<222> (1)..(1)
<223> glycated

<220>
<221> MISC_FEATURE
<222> (2)..(2)
<223> Cysteine may be alkylated

<220>

<221> MISC_FEATURE
<222> (11)..(11)
<223> Cysteine may be alkylated

<400> 23

```
Lys Cys Ser Thr Ser Ser Leu Leu Glu Ala Cys Thr Phe Arg
1               5                   10
```

<210> 24
<211> 17
<212> PRT
<213> Homo sapiens

<220>
<221> MISC_FEATURE
<222> (7)..(7)
<223> glycated

<400> 24

```
Ala Asp Ser Ser Pro Val Lys Ala Gly Val Glu Thr Thr Thr Pro Ser
1               5                   10                  15

Lys
```

<210> 25
<211> 12
<212> PRT
<213> Homo sapiens

<220>
<221> MISC_FEATURE
<222> (2)..(2)
<223> glycated

<400> 25

```
Ala Lys Val Gln Pro Tyr Leu Asp Asp Phe Gln Lys
1               5                   10
```

<210> 26
<211> 10
<212> PRT
<213> Homo sapiens

<220>
<221> MISC_FEATURE
<222> (1)..(1)
<223> glycated

<220>
<221> MISC_FEATURE
<222> (6)..(6)
<223> Methionine may be oxidized

<400> 26

```
        Lys Trp Gln Glu Glu Met Glu Leu Tyr Arg
        1               5                   10
```

<210> 27
<211> 17
<212> PRT
<213> Homo sapiens

<220>
<221> MISC_FEATURE
<222> (5)..(5)
<223> glycated

<220>
<221> MISC_FEATURE
<222> (9)..(9)
<223> Cysteine may be alkylated

<220>
<221> MISC_FEATURE
<222> (13)..(13)
<223> Cysteine may be alkylated

<400> 27

```
        Ala Val Gly Asp Lys Leu Pro Glu Cys Glu Ala Val Cys Gly Lys Pro
        1               5                   10                  15

        Lys
```

<210> 28
<211> 17
<212> PRT
<213> Homo sapiens

<220>
<221> MISC_FEATURE
<222> (5)..(5)
<223> glycated

<400> 28

```
        Ser Glu Glu Thr Lys Glu Asn Glu Gly Phe Thr Val Thr Ala Glu Gly
        1               5                   10                  15

        Lys
```

<210> 29
<211> 15
<212> PRT
<213> Homo sapiens

<220>
<221> MISC_FEATURE
<222> (1)..(1)
<223> Methionine may be oxidized

```
<220>
<221> MISC_FEATURE
<222> (2)..(2)
<223> glycated

<400> 29


        Met Lys Gly Leu Ile Asp Glu Val Asn Gln Asp Phe Thr Asn Arg
        1               5                   10                  15


<210> 30
<211> 16
<212> PRT
<213> Homo sapiens


<220>
<221> MISC_FEATURE
<222> (6)..(6)
<223> glycated

<400> 30


        Ser Ser Ser Tyr Ser Lys Gln Phe Thr Ser Ser Thr Ser Tyr Asn Arg
        1               5                   10                  15


<210> 31
<211> 609
<212> PRT
<213> Homo sapiens


<400> 31
```

Met Lys Trp Val Thr Phe Ile Ser Leu Leu Phe Leu Phe Ser Ser Ala
1 5 10 15

Tyr Ser Arg Gly Val Phe Arg Arg Asp Ala His Lys Ser Glu Val Ala
20 25 30

His Arg Phe Lys Asp Leu Gly Glu Glu Asn Phe Lys Ala Leu Val Leu
35 40 45

Ile Ala Phe Ala Gln Tyr Leu Gln Gln Cys Pro Phe Glu Asp His Val
50 55 60

Lys Leu Val Asn Glu Val Thr Glu Phe Ala Lys Thr Cys Val Ala Asp
65 70 75 80

Glu Ser Ala Glu Asn Cys Asp Lys Ser Leu His Thr Leu Phe Gly Asp
85 90 95

Lys Leu Cys Thr Val Ala Thr Leu Arg Glu Thr Tyr Gly Glu Met Ala
100 105 110

Asp Cys Cys Ala Lys Gln Glu Pro Glu Arg Asn Glu Cys Phe Leu Gln
115 120 125

His Lys Asp Asp Asn Pro Asn Leu Pro Arg Leu Val Arg Pro Glu Val
130 135 140

Asp Val Met Cys Thr Ala Phe His Asp Asn Glu Glu Thr Phe Leu Lys

**27**

145            150            155            160

Lys Tyr Leu Tyr Glu Ile Ala Arg Arg His Pro Tyr Phe Tyr Ala Pro
                165                 170                 175

Glu Leu Leu Phe Phe Ala Lys Arg Tyr Lys Ala Ala Phe Thr Glu Cys
            180                 185                 190

Cys Gln Ala Ala Asp Lys Ala Ala Cys Leu Leu Pro Lys Leu Asp Glu
            195                 200                 205

Leu Arg Asp Glu Gly Lys Ala Ser Ser Ala Lys Gln Arg Leu Lys Cys
    210                 215                 220

Ala Ser Leu Gln Lys Phe Gly Glu Arg Ala Phe Lys Ala Trp Ala Val
225                 230                 235                 240

Ala Arg Leu Ser Gln Arg Phe Pro Lys Ala Glu Phe Ala Glu Val Ser
            245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Ile
        275                 280                 285

Cys Glu Asn Gln Asp Ser Ile Ser Ser Lys Leu Lys Glu Cys Cys Glu
    290                 295                 300

Lys Pro Leu Leu Glu Lys Ser His Cys Ile Ala Glu Val Glu Asn Asp
305                 310                 315                 320

Glu Met Pro Ala Asp Leu Pro Ser Leu Ala Ala Asp Phe Val Glu Ser
            325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Val
        355                 360                 365

Leu Leu Leu Arg Leu Ala Lys Thr Tyr Glu Thr Thr Leu Glu Lys Cys
    370                 375                 380

Cys Ala Ala Ala Asp Pro His Glu Cys Tyr Ala Lys Val Phe Asp Glu
385                 390                 395                 400

```
Phe Lys Pro Leu Val Glu Glu Pro Gln Asn Leu Ile Lys Gln Asn Cys
            405             410             415

Glu Leu Phe Glu Gln Leu Gly Glu Tyr Lys Phe Gln Asn Ala Leu Leu
            420             425             430

Val Arg Tyr Thr Lys Lys Val Pro Gln Val Ser Thr Pro Thr Leu Val
            435             440             445

Glu Val Ser Arg Asn Leu Gly Lys Val Gly Ser Lys Cys Cys Lys His
            450             455             460

Pro Glu Ala Lys Arg Met Pro Cys Ala Glu Asp Tyr Leu Ser Val Val
465             470             475             480

Leu Asn Gln Leu Cys Val Leu His Glu Lys Thr Pro Val Ser Asp Arg
            485             490             495

Val Thr Lys Cys Cys Thr Glu Ser Leu Val Asn Arg Arg Pro Cys Phe
            500             505             510

Ser Ala Leu Glu Val Asp Glu Thr Tyr Val Pro Lys Glu Phe Asn Ala
            515             520             525

Glu Thr Phe Thr Phe His Ala Asp Ile Cys Thr Leu Ser Glu Lys Glu
            530             535             540

Arg Gln Ile Lys Lys Gln Thr Ala Leu Val Glu Leu Val Lys His Lys
545             550             555             560

Pro Lys Ala Thr Lys Glu Gln Leu Lys Ala Val Met Asp Asp Phe Ala
            565             570             575

Ala Phe Val Glu Lys Cys Cys Lys Ala Asp Asp Lys Glu Thr Cys Phe
            580             585             590

Ala Glu Glu Gly Lys Lys Leu Val Ala Ala Ser Gln Ala Ala Leu Gly
            595             600             605

Leu
```

<210> 32
<211> 106
<212> PRT
<213> Homo sapiens

<400> 32

```
Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
            85                  90                  95

Val Thr Lys Ser Phe Asn Arg Gly Glu Cys
            100                 105
```

<210> 33
<211> 491
<212> PRT
<213> Homo sapiens

<400> 33

```
Met Lys Arg Met Val Ser Trp Ser Phe His Lys Leu Lys Thr Met Lys
1               5                   10                  15

His Leu Leu Leu Leu Leu Leu Cys Val Phe Leu Val Lys Ser Gln Gly
            20                  25                  30

Val Asn Asp Asn Glu Glu Gly Phe Phe Ser Ala Arg Gly His Arg Pro
        35                  40                  45

Leu Asp Lys Lys Arg Glu Glu Ala Pro Ser Leu Arg Pro Ala Pro Pro
    50                  55                  60

Pro Ile Ser Gly Gly Gly Tyr Arg Ala Arg Pro Ala Lys Ala Ala Ala
65                  70                  75                  80

Thr Gln Lys Lys Val Glu Arg Lys Ala Pro Asp Ala Gly Gly Cys Leu
            85                  90                  95

His Ala Asp Pro Asp Leu Gly Val Leu Cys Pro Thr Gly Cys Gln Leu
            100                 105                 110
```

```
Gln Glu Ala Leu Leu Gln Gln Glu Arg Pro Ile Arg Asn Ser Val Asp
        115                 120             125

Glu Leu Asn Asn Asn Val Glu Ala Val Ser Gln Thr Ser Ser Ser Ser
        130                 135             140

Phe Gln Tyr Met Tyr Leu Leu Lys Asp Leu Trp Gln Lys Arg Gln Lys
145                 150             155                 160

Gln Val Lys Asp Asn Glu Asn Val Val Asn Glu Tyr Ser Ser Glu Leu
                165                 170             175

Glu Lys His Gln Leu Tyr Ile Asp Glu Thr Val Asn Ser Asn Ile Pro
            180                 185             190

Thr Asn Leu Arg Val Leu Arg Ser Ile Leu Glu Asn Leu Arg Ser Lys
            195                 200             205

Ile Gln Lys Leu Glu Ser Asp Val Ser Ala Gln Met Glu Tyr Cys Arg
        210                 215             220

Thr Pro Cys Thr Val Ser Cys Asn Ile Pro Val Val Ser Gly Lys Glu
225                 230                 235                 240

Cys Glu Glu Ile Ile Arg Lys Gly Gly Glu Thr Ser Glu Met Tyr Leu
                245                 250             255

Ile Gln Pro Asp Ser Ser Val Lys Pro Tyr Arg Val Tyr Cys Asp Met
            260                 265             270

Asn Thr Glu Asn Gly Gly Trp Thr Val Ile Gln Asn Arg Gln Asp Gly
            275                 280             285

Ser Val Asp Phe Gly Arg Lys Trp Asp Pro Tyr Lys Gln Gly Phe Gly
        290                 295             300

Asn Val Ala Thr Asn Thr Asp Gly Lys Asn Tyr Cys Gly Leu Pro Gly
305                 310                 315                 320

Glu Tyr Trp Leu Gly Asn Asp Lys Ile Ser Gln Leu Thr Arg Met Gly
                325                 330             335

Pro Thr Glu Leu Leu Ile Glu Met Glu Asp Trp Lys Gly Asp Lys Val
            340                 345             350

Lys Ala His Tyr Gly Gly Phe Thr Val Gln Asn Glu Ala Asn Lys Tyr
            355                 360             365
```

31

```
Gln Ile Ser Val Asn Lys Tyr Arg Gly Thr Ala Gly Asn Ala Leu Met
    370             375             380

Asp Gly Ala Ser Gln Leu Met Gly Glu Asn Arg Thr Met Thr Ile His
385             390             395             400

Asn Gly Met Phe Phe Ser Thr Tyr Asp Arg Asp Asn Asp Gly Trp Leu
                405             410             415

Thr Ser Asp Pro Arg Lys Gln Cys Ser Lys Glu Asp Gly Gly Gly Trp
            420             425             430

Trp Tyr Asn Arg Cys His Ala Ala Asn Pro Asn Gly Arg Tyr Tyr Trp
        435             440             445

Gly Gly Gln Tyr Thr Trp Asp Met Ala Lys His Gly Thr Asp Asp Gly
    450             455             460

Val Val Trp Met Asn Trp Lys Gly Ser Trp Tyr Ser Met Arg Lys Met
465             470             475             480

Ser Met Lys Ile Arg Pro Phe Phe Pro Gln Gln
            485             490
```

<210> 34
<211> 1474
<212> PRT
<213> Homo sapiens

<400> 34

```
Met Gly Lys Asn Lys Leu Leu His Pro Ser Leu Val Leu Leu Leu Leu
1               5               10              15

Val Leu Leu Pro Thr Asp Ala Ser Val Ser Gly Lys Pro Gln Tyr Met
            20              25              30

Val Leu Val Pro Ser Leu Leu His Thr Glu Thr Thr Glu Lys Gly Cys
        35              40              45

Val Leu Leu Ser Tyr Leu Asn Glu Thr Val Thr Val Ser Ala Ser Leu
    50              55              60

Glu Ser Val Arg Gly Asn Arg Ser Leu Phe Thr Asp Leu Glu Ala Glu
65              70              75              80

Asn Asp Val Leu His Cys Val Ala Phe Ala Val Pro Lys Ser Ser Ser
            85              90              95
```

32

Asn Glu Glu Val Met Phe Leu Thr Val Gln Val Lys Gly Pro Thr Gln
100                     105                 110

Glu Phe Lys Lys Arg Thr Thr Val Met Val Lys Asn Glu Asp Ser Leu
115                     120                 125

Val Phe Val Gln Thr Asp Lys Ser Ile Tyr Lys Pro Gly Gln Thr Val
130                     135                 140

Lys Phe Arg Val Val Ser Met Asp Glu Asn Phe His Pro Leu Asn Glu
145                 150                 155                 160

Leu Ile Pro Leu Val Tyr Ile Gln Asp Pro Lys Gly Asn Arg Ile Ala
                165                 170                 175

Gln Trp Gln Ser Phe Gln Leu Glu Gly Gly Leu Lys Gln Phe Ser Phe
                180                 185                 190

Pro Leu Ser Ser Glu Pro Phe Gln Gly Ser Tyr Lys Val Val Val Gln
                195                 200                 205

Lys Lys Ser Gly Gly Arg Thr Glu His Pro Phe Thr Val Glu Glu Phe
210                 215                 220

Val Leu Pro Lys Phe Glu Val Gln Val Thr Val Pro Lys Ile Ile Thr
225                 230                 235                 240

Ile Leu Glu Glu Glu Met Asn Val Ser Val Cys Gly Leu Tyr Thr Tyr
                245                 250                 255

Gly Lys Pro Val Pro Gly His Val Thr Val Ser Ile Cys Arg Lys Tyr
                260                 265                 270

Ser Asp Ala Ser Asp Cys His Gly Glu Asp Ser Gln Ala Phe Cys Glu
                275                 280                 285

Lys Phe Ser Gly Gln Leu Asn Ser His Gly Cys Phe Tyr Gln Gln Val
                290                 295                 300

Lys Thr Lys Val Phe Gln Leu Lys Arg Lys Glu Tyr Glu Met Lys Leu
305                 310                 315                 320

His Thr Glu Ala Gln Ile Gln Glu Glu Gly Thr Val Val Glu Leu Thr
                325                 330                 335

Gly Arg Gln Ser Ser Glu Ile Thr Arg Thr Ile Thr Lys Leu Ser Phe
                340                 345                 350

```
Val Lys Val Asp Ser His Phe Arg Gln Gly Ile Pro Phe Phe Gly Gln
    355                 360             365

Val Arg Leu Val Asp Gly Lys Gly Val Pro Ile Pro Asn Lys Val Ile
    370             375             380

Phe Ile Arg Gly Asn Glu Ala Asn Tyr Tyr Ser Asn Ala Thr Thr Asp
385                 390             395                 400

Glu His Gly Leu Val Gln Phe Ser Ile Asn Thr Thr Asn Val Met Gly
                405             410             415

Thr Ser Leu Thr Val Arg Val Asn Tyr Lys Asp Arg Ser Pro Cys Tyr
            420             425             430

Gly Tyr Gln Trp Val Ser Glu Glu His Glu Glu Ala His His Thr Ala
        435             440             445

Tyr Leu Val Phe Ser Pro Ser Lys Ser Phe Val His Leu Glu Pro Met
    450             455             460

Ser His Glu Leu Pro Cys Gly His Thr Gln Thr Val Gln Ala His Tyr
465             470             475             480

Ile Leu Asn Gly Gly Thr Leu Leu Gly Leu Lys Lys Leu Ser Phe Tyr
                485             490             495

Tyr Leu Ile Met Ala Lys Gly Gly Ile Val Arg Thr Gly Thr His Gly
            500             505             510

Leu Leu Val Lys Gln Glu Asp Met Lys Gly His Phe Ser Ile Ser Ile
            515             520             525

Pro Val Lys Ser Asp Ile Ala Pro Val Ala Arg Leu Leu Ile Tyr Ala
    530             535             540

Val Leu Pro Thr Gly Asp Val Ile Gly Asp Ser Ala Lys Tyr Asp Val
545             550             555             560

Glu Asn Cys Leu Ala Asn Lys Val Asp Leu Ser Phe Ser Pro Ser Gln
                565             570             575

Ser Leu Pro Ala Ser His Ala His Leu Arg Val Thr Ala Ala Pro Gln
            580             585             590

Ser Val Cys Ala Leu Arg Ala Val Asp Gln Ser Val Leu Leu Met Lys
```

```
                   595                    600                    605


        Pro Asp Ala Glu Leu Ser Ala Ser Ser Val Tyr Asn Leu Leu Pro Glu
            610             615             620


        Lys Asp Leu Thr Gly Phe Pro Gly Pro Leu Asn Asp Gln Asp Asn Glu
        625             630             635             640


        Asp Cys Ile Asn Arg His Asn Val Tyr Ile Asn Gly Ile Thr Tyr Thr
                        645             650             655


        Pro Val Ser Ser Thr Asn Glu Lys Asp Met Tyr Ser Phe Leu Glu Asp
                    660             665             670


        Met Gly Leu Lys Ala Phe Thr Asn Ser Lys Ile Arg Lys Pro Lys Met
                675             680             685


        Cys Pro Gln Leu Gln Gln Tyr Glu Met His Gly Pro Glu Gly Leu Arg
            690             695             700


        Val Gly Phe Tyr Glu Ser Asp Val Met Gly Arg Gly His Ala Arg Leu
        705             710             715             720


        Val His Val Glu Glu Pro His Thr Glu Thr Val Arg Lys Tyr Phe Pro
                    725             730             735


        Glu Thr Trp Ile Trp Asp Leu Val Val Val Asn Ser Ala Gly Val Ala
                740             745             750


        Glu Val Gly Val Thr Val Pro Asp Thr Ile Thr Glu Trp Lys Ala Gly
                755             760             765


        Ala Phe Cys Leu Ser Glu Asp Ala Gly Leu Gly Ile Ser Ser Thr Ala
            770             775             780


        Ser Leu Arg Ala Phe Gln Pro Phe Phe Val Glu Leu Thr Met Pro Tyr
        785             790             795             800


        Ser Val Ile Arg Gly Glu Ala Phe Thr Leu Lys Ala Thr Val Leu Asn
                        805             810             815


        Tyr Leu Pro Lys Cys Ile Arg Val Ser Val Gln Leu Glu Ala Ser Pro
                    820             825             830


        Ala Phe Leu Ala Val Pro Val Glu Lys Glu Gln Ala Pro His Cys Ile
            835             840             845
```

35

```
Cys Ala Asn Gly Arg Gln Thr Val Ser Trp Ala Val Thr Pro Lys Ser
    850                 855                 860

Leu Gly Asn Val Asn Phe Thr Val Ser Ala Glu Ala Leu Glu Ser Gln
    865                 870                 875                 880

Glu Leu Cys Gly Thr Glu Val Pro Ser Val Pro Glu His Gly Arg Lys
                885                 890                 895

Asp Thr Val Ile Lys Pro Leu Leu Val Glu Pro Glu Gly Leu Glu Lys
                900                 905                 910

Glu Thr Thr Phe Asn Ser Leu Leu Cys Pro Ser Gly Gly Glu Val Ser
        915                 920                 925

Glu Glu Leu Ser Leu Lys Leu Pro Pro Asn Val Val Glu Glu Ser Ala
    930                 935                 940

Arg Ala Ser Val Ser Val Leu Gly Asp Ile Leu Gly Ser Ala Met Gln
945                 950                 955                 960

Asn Thr Gln Asn Leu Leu Gln Met Pro Tyr Gly Cys Gly Glu Gln Asn
                965                 970                 975

Met Val Leu Phe Ala Pro Asn Ile Tyr Val Leu Asp Tyr Leu Asn Glu
                980                 985                 990

Thr Gln Gln Leu Thr Pro Glu Ile  Lys Ser Lys Ala Ile  Gly Tyr Leu
            995             1000                1005

Asn Thr  Gly Tyr Gln Arg Gln  Leu Asn Tyr Lys His  Tyr Asp Gly
    1010                1015                1020

Ser Tyr  Ser Thr Phe Gly Glu  Arg Tyr Gly Arg Asn  Gln Gly Asn
    1025                1030                1035

Thr Trp  Leu Thr Ala Phe Val  Leu Lys Thr Phe Ala  Gln Ala Arg
    1040                1045                1050

Ala Tyr  Ile Phe Ile Asp Glu  Ala His Ile Thr Gln  Ala Leu Ile
    1055                1060                1065

Trp Leu  Ser Gln Arg Gln Lys  Asp Asn Gly Cys Phe  Arg Ser Ser
    1070                1075                1080

Gly Ser  Leu Leu Asn Asn Ala  Ile Lys Gly Gly Val  Glu Asp Glu
    1085                1090                1095
```

36

```
Val Thr  Leu Ser Ala Tyr Ile  Thr Ile Ala Leu Leu  Glu Ile Pro
    1100             1105             1110

Leu Thr  Val Thr His Pro Val  Val Arg Asn Ala Leu  Phe Cys Leu
    1115             1120             1125

Glu Ser  Ala Trp Lys Thr Ala  Gln Glu Gly Asp His  Gly Ser His
    1130             1135             1140

Val Tyr  Thr Lys Ala Leu Leu  Ala Tyr Ala Phe Ala  Leu Ala Gly
    1145             1150             1155

Asn Gln  Asp Lys Arg Lys Glu  Val Leu Lys Ser Leu  Asn Glu Glu
    1160             1165             1170

Ala Val  Lys Lys Asp Asn Ser  Val His Trp Glu Arg  Pro Gln Lys
    1175             1180             1185

Pro Lys  Ala Pro Val Gly His  Phe Tyr Glu Pro Gln  Ala Pro Ser
    1190             1195             1200

Ala Glu  Val Glu Met Thr Ser  Tyr Val Leu Leu Ala  Tyr Leu Thr
    1205             1210             1215

Ala Gln  Pro Ala Pro Thr Ser  Glu Asp Leu Thr Ser  Ala Thr Asn
    1220             1225             1230

Ile Val  Lys Trp Ile Thr Lys  Gln Gln Asn Ala Gln  Gly Gly Phe
    1235             1240             1245

Ser Ser  Thr Gln Asp Thr Val  Val Ala Leu His Ala  Leu Ser Lys
    1250             1255             1260

Tyr Gly  Ala Ala Thr Phe Thr  Arg Thr Gly Lys Ala  Ala Gln Val
    1265             1270             1275

Thr Ile  Gln Ser Ser Gly Thr  Phe Ser Ser Lys Phe  Gln Val Asp
    1280             1285             1290

Asn Asn  Asn Arg Leu Leu Leu  Gln Gln Val Ser Leu  Pro Glu Leu
    1295             1300             1305

Pro Gly  Glu Tyr Ser Met Lys  Val Thr Gly Glu Gly  Cys Val Tyr
    1310             1315             1320

Leu Gln  Thr Ser Leu Lys Tyr  Asn Ile Leu Pro Glu  Lys Glu Glu
    1325             1330             1335
```

37

```
Phe Pro Phe Ala Leu Gly Val   Gln Thr Leu Pro Gln   Thr Cys Asp
    1340                1345              1350

Glu Pro Lys Ala His Thr Ser   Phe Gln Ile Ser Leu   Ser Val Ser
    1355                1360              1365

Tyr Thr Gly Ser Arg Ser Ala   Ser Asn Met Ala Ile   Val Asp Val
    1370                1375              1380

Lys Met Val Ser Gly Phe Ile   Pro Leu Lys Pro Thr   Val Lys Met
    1385                1390              1395

Leu Glu Arg Ser Asn His Val   Ser Arg Thr Glu Val   Ser Ser Asn
    1400                1405              1410

His Val Leu Ile Tyr Leu Asp   Lys Val Ser Asn Gln   Thr Leu Ser
    1415                1420              1425

Leu Phe Phe Thr Val Leu Gln   Asp Val Pro Val Arg   Asp Leu Lys
    1430                1435              1440

Pro Ala Ile Val Lys Val Tyr   Asp Tyr Tyr Glu Thr   Asp Glu Phe
    1445                1450              1455

Ala Ile Ala Glu Tyr Asn Ala   Pro Cys Ser Lys Asp   Leu Gly Asn
    1460                1465              1470

Ala
```

<210> 35
<211> 698
<212> PRT
<213> Homo sapiens

<400> 35

```
Met Arg Leu Ala Val Gly Ala Leu Leu Val Cys Ala Val Leu Gly Leu
1                   5                   10                  15

Cys Leu Ala Val Pro Asp Lys Thr Val Arg Trp Cys Ala Val Ser Glu
              20                  25                  30

His Glu Ala Thr Lys Cys Gln Ser Phe Arg Asp His Met Lys Ser Val
          35                  40                  45

Ile Pro Ser Asp Gly Pro Ser Val Ala Cys Val Lys Lys Ala Ser Tyr
      50                  55                  60
```

38

Leu Asp Cys Ile Arg Ala Ile Ala Ala Asn Glu Ala Asp Ala Val Thr
65                  70              75                  80


Leu Asp Ala Gly Leu Val Tyr Asp Ala Tyr Leu Ala Pro Asn Asn Leu
                85                  90                  95


Lys Pro Val Val Ala Glu Phe Tyr Gly Ser Lys Glu Asp Pro Gln Thr
                100                 105                 110


Phe Tyr Tyr Ala Val Ala Val Val Lys Lys Asp Ser Gly Phe Gln Met
            115                 120                 125


Asn Gln Leu Arg Gly Lys Lys Ser Cys His Thr Gly Leu Gly Arg Ser
    130                 135                 140


Ala Gly Trp Asn Ile Pro Ile Gly Leu Leu Tyr Cys Asp Leu Pro Glu
145                 150                 155                 160


Pro Arg Lys Pro Leu Glu Lys Ala Val Ala Asn Phe Phe Ser Gly Ser
                165                 170                 175


Cys Ala Pro Cys Ala Asp Gly Thr Asp Phe Pro Gln Leu Cys Gln Leu
            180                 185                 190


Cys Pro Gly Cys Gly Cys Ser Thr Leu Asn Gln Tyr Phe Gly Tyr Ser
            195                 200                 205


Gly Ala Phe Lys Cys Leu Lys Asp Gly Ala Gly Asp Val Ala Phe Val
    210                 215                 220


Lys His Ser Thr Ile Phe Glu Asn Leu Ala Asn Lys Ala Asp Arg Asp
225                 230                 235                 240


Gln Tyr Glu Leu Leu Cys Leu Asp Asn Thr Arg Lys Pro Val Asp Glu
                245                 250                 255


Tyr Lys Asp Cys His Leu Ala Gln Val Pro Ser His Thr Val Val Ala
            260                 265                 270


Arg Ser Met Gly Gly Lys Glu Asp Leu Ile Trp Glu Leu Leu Asn Gln
        275                 280                 285


Ala Gln Glu His Phe Gly Lys Asp Lys Ser Lys Glu Phe Gln Leu Phe
    290                 295                 300


Ser Ser Pro His Gly Lys Asp Leu Leu Phe Lys Asp Ser Ala His Gly

39

|  | 305 |  |  | 310 |  |  | 315 |  |  | 320 |

Phe Leu Lys Val Pro Pro Arg Met Asp Ala Lys Met Tyr Leu Gly Tyr
325 330 335

Glu Tyr Val Thr Ala Ile Arg Asn Leu Arg Glu Gly Thr Cys Pro Glu
340 345 350

Ala Pro Thr Asp Glu Cys Lys Pro Val Lys Trp Cys Ala Leu Ser His
355 360 365

His Glu Arg Leu Lys Cys Asp Glu Trp Ser Val Asn Ser Val Gly Lys
370 375 380

Ile Glu Cys Val Ser Ala Glu Thr Thr Glu Asp Cys Ile Ala Lys Ile
385 390 395 400

Met Asn Gly Glu Ala Asp Ala Met Ser Leu Asp Gly Gly Phe Val Tyr
405 410 415

Ile Ala Gly Lys Cys Gly Leu Val Pro Val Leu Ala Glu Asn Tyr Asn
420 425 430

Lys Ser Asp Asn Cys Glu Asp Thr Pro Glu Ala Gly Tyr Phe Ala Ile
435 440 445

Ala Val Val Lys Lys Ser Ala Ser Asp Leu Thr Trp Asp Asn Leu Lys
450 455 460

Gly Lys Lys Ser Cys His Thr Ala Val Gly Arg Thr Ala Gly Trp Asn
465 470 475 480

Ile Pro Met Gly Leu Leu Tyr Asn Lys Ile Asn His Cys Arg Phe Asp
485 490 495

Glu Phe Phe Ser Glu Gly Cys Ala Pro Gly Ser Lys Lys Asp Ser Ser
500 505 510

Leu Cys Lys Leu Cys Met Gly Ser Gly Leu Asn Leu Cys Glu Pro Asn
515 520 525

Asn Lys Glu Gly Tyr Tyr Gly Tyr Thr Gly Ala Phe Arg Cys Leu Val
530 535 540

Glu Lys Gly Asp Val Ala Phe Val Lys His Gln Thr Val Pro Gln Asn
545 550 555 560

```
Thr Gly Gly Lys Asn Pro Asp Pro Trp Ala Lys Asn Leu Asn Glu Lys
              565               570               575

Asp Tyr Glu Leu Leu Cys Leu Asp Gly Thr Arg Lys Pro Val Glu Glu
              580               585               590

Tyr Ala Asn Cys His Leu Ala Arg Ala Pro Asn His Ala Val Val Thr
              595               600               605

Arg Lys Asp Lys Glu Ala Cys Val His Lys Ile Leu Arg Gln Gln Gln
    610               615               620

His Leu Phe Gly Ser Asn Val Thr Asp Cys Ser Gly Asn Phe Cys Leu
625               630               635               640

Phe Arg Ser Glu Thr Lys Asp Leu Leu Phe Arg Asp Asp Thr Val Cys
              645               650               655

Leu Ala Lys Leu His Asp Arg Asn Thr Tyr Glu Lys Tyr Leu Gly Glu
              660               665               670

Glu Tyr Val Lys Ala Val Gly Asn Leu Arg Lys Cys Ser Thr Ser Ser
              675               680               685

Leu Leu Glu Ala Cys Thr Phe Arg Arg Pro
              690               695
```

<210> 36
<211> 106
<212> PRT
<213> Homo sapiens

<400> 36

```
Gly Gln Pro Lys Ala Asn Pro Thr Val Thr Leu Phe Pro Pro Ser Ser
1               5               10              15

Glu Glu Leu Gln Ala Asn Lys Ala Thr Leu Val Cys Leu Ile Ser Asp
              20              25              30

Phe Tyr Pro Gly Ala Val Thr Val Ala Trp Lys Ala Asp Gly Ser Pro
              35              40              45

Val Lys Ala Gly Val Glu Thr Thr Lys Pro Ser Lys Gln Ser Asn Asn
    50              55              60

Lys Tyr Ala Ala Ser Ser Tyr Leu Ser Leu Thr Pro Glu Gln Trp Lys
65              70              75              80
```

```
          Ser His Arg Ser Tyr Ser Cys Gln Val Thr His Glu Gly Ser Thr Val
                          85              90                  95


          Glu Lys Thr Val Ala Pro Thr Glu Cys Ser
                      100             105
```

<210> 37
<211> 267
<212> PRT
<213> Homo sapiens

<400> 37

```
          Met Lys Ala Ala Val Leu Thr Leu Ala Val Leu Phe Leu Thr Gly Ser
          1               5               10              15


          Gln Ala Arg His Phe Trp Gln Gln Asp Glu Pro Pro Gln Ser Pro Trp
                      20              25              30


          Asp Arg Val Lys Asp Leu Ala Thr Val Tyr Val Asp Val Leu Lys Asp
                      35              40              45


          Ser Gly Arg Asp Tyr Val Ser Gln Phe Glu Gly Ser Ala Leu Gly Lys
                  50              55              60


          Gln Leu Asn Leu Lys Leu Leu Asp Asn Trp Asp Ser Val Thr Ser Thr
          65              70              75              80


          Phe Ser Lys Leu Arg Glu Gln Leu Gly Pro Val Thr Gln Glu Phe Trp
                          85              90                  95


          Asp Asn Leu Glu Lys Glu Thr Glu Gly Leu Arg Gln Glu Met Ser Lys
                      100             105             110


          Asp Leu Glu Glu Val Lys Ala Lys Val Gln Pro Tyr Leu Asp Asp Phe
                      115             120             125


          Gln Lys Lys Trp Gln Glu Glu Met Glu Leu Tyr Arg Gln Lys Val Glu
                  130             135             140


          Pro Leu Arg Ala Glu Leu Gln Glu Gly Ala Arg Gln Lys Leu His Glu
          145             150             155             160


          Leu Gln Glu Lys Leu Ser Pro Leu Gly Glu Glu Met Arg Asp Arg Ala
                          165             170             175


          Arg Ala His Val Asp Ala Leu Arg Thr His Leu Ala Pro Tyr Ser Asp
                      180             185             190
```

42

```
Glu Leu Arg Gln Arg Leu Ala Ala Arg Leu Glu Ala Leu Lys Glu Asn
        195             200             205

Gly Gly Ala Arg Leu Ala Glu Tyr His Ala Lys Ala Thr Glu His Leu
        210             215             220

Ser Thr Leu Ser Glu Lys Ala Lys Pro Ala Leu Glu Asp Leu Arg Gln
225             230             235             240

Gly Leu Leu Pro Val Leu Glu Ser Phe Lys Val Ser Phe Leu Ser Ala
            245             250             255

Leu Glu Glu Tyr Thr Lys Lys Leu Asn Thr Gln
            260             265
```

<210> 38
<211> 406
<212> PRT
<213> Homo sapiens

<400> 38

```
Met Ser Ala Leu Gly Ala Val Ile Ala Leu Leu Leu Trp Gly Gln Leu
1               5               10              15

Phe Ala Val Asp Ser Gly Asn Asp Val Thr Asp Ile Ala Asp Asp Gly
            20              25              30

Cys Pro Lys Pro Pro Glu Ile Ala His Gly Tyr Val Glu His Ser Val
        35              40              45

Arg Tyr Gln Cys Lys Asn Tyr Tyr Lys Leu Arg Thr Glu Gly Asp Gly
        50              55              60

Val Tyr Thr Leu Asn Asp Lys Lys Gln Trp Ile Asn Lys Ala Val Gly
65              70              75              80

Asp Lys Leu Pro Glu Cys Glu Ala Asp Asp Gly Cys Pro Lys Pro Pro
            85              90              95

Glu Ile Ala His Gly Tyr Val Glu His Ser Val Arg Tyr Gln Cys Lys
            100             105             110

Asn Tyr Tyr Lys Leu Arg Thr Glu Gly Asp Gly Val Tyr Thr Leu Asn
        115             120             125

Asn Glu Lys Gln Trp Ile Asn Lys Ala Val Gly Asp Lys Leu Pro Glu
        130             135             140
```

43

```
Cys Glu Ala Val Cys Gly Lys Pro Lys Asn Pro Ala Asn Pro Val Gln
145                 150             155                 160

Arg Ile Leu Gly Gly His Leu Asp Ala Lys Gly Ser Phe Pro Trp Gln
                165                 170                 175

Ala Lys Met Val Ser His His Asn Leu Thr Thr Gly Ala Thr Leu Ile
            180                 185                 190

Asn Glu Gln Trp Leu Leu Thr Thr Ala Lys Asn Leu Phe Leu Asn His
            195                 200                 205

Ser Glu Asn Ala Thr Ala Lys Asp Ile Ala Pro Thr Leu Thr Leu Tyr
            210                 215                 220

Val Gly Lys Lys Gln Leu Val Glu Ile Glu Lys Val Val Leu His Pro
225                 230                 235                 240

Asn Tyr Ser Gln Val Asp Ile Gly Leu Ile Lys Leu Lys Gln Lys Val
                245                 250                 255

Ser Val Asn Glu Arg Val Met Pro Ile Cys Leu Pro Ser Lys Asp Tyr
            260                 265                 270

Ala Glu Val Gly Arg Val Gly Tyr Val Ser Gly Trp Gly Arg Asn Ala
            275                 280                 285

Asn Phe Lys Phe Thr Asp His Leu Lys Tyr Val Met Leu Pro Val Ala
    290                 295                 300

Asp Gln Asp Gln Cys Ile Arg His Tyr Glu Gly Ser Thr Val Pro Glu
305                 310                 315                 320

Lys Lys Thr Pro Lys Ser Pro Val Gly Val Gln Pro Ile Leu Asn Glu
                325                 330                 335

His Thr Phe Cys Ala Gly Met Ser Lys Tyr Gln Glu Asp Thr Cys Tyr
            340                 345                 350

Gly Asp Ala Gly Ser Ala Phe Ala Val His Asp Leu Glu Glu Asp Thr
            355                 360                 365

Trp Tyr Ala Thr Gly Ile Leu Ser Phe Asp Lys Ser Cys Ala Val Ala
    370                 375                 380

Glu Tyr Gly Val Tyr Val Lys Val Thr Ser Ile Gln Asp Trp Val Gln
385                 390                 395                 400
```

```
                    Lys Thr Ile Ala Glu Asn
                                    405
```

<210> 39
<211> 1663
<212> PRT
<213> Homo sapiens


<400> 39

```
        Met Gly Pro Thr Ser Gly Pro Ser Leu Leu Leu Leu Leu Leu Thr His
        1               5                   10                  15


        Leu Pro Leu Ala Leu Gly Ser Pro Met Tyr Ser Ile Ile Thr Pro Asn
                        20                  25                  30


        Ile Leu Arg Leu Glu Ser Glu Glu Thr Met Val Leu Glu Ala His Asp
                    35                  40                  45


        Ala Gln Gly Asp Val Pro Val Thr Val Thr Val His Asp Phe Pro Gly
                50                  55                  60


        Lys Lys Leu Val Leu Ser Ser Glu Lys Thr Val Leu Thr Pro Ala Thr
        65                  70                  75                  80


        Asn His Met Gly Asn Val Thr Phe Thr Ile Pro Ala Asn Arg Glu Phe
                            85                  90                  95


        Lys Ser Glu Lys Gly Arg Asn Lys Phe Val Thr Val Gln Ala Thr Phe
                        100                 105                 110


        Gly Thr Gln Val Val Glu Lys Val Val Leu Val Ser Leu Gln Ser Gly
                    115                 120                 125


        Tyr Leu Phe Ile Gln Thr Asp Lys Thr Ile Tyr Thr Pro Gly Ser Thr
                130                 135                 140


        Val Leu Tyr Arg Ile Phe Thr Val Asn His Lys Leu Leu Pro Val Gly
        145                 150                 155                 160


        Arg Thr Val Met Val Asn Ile Glu Asn Pro Glu Gly Ile Pro Val Lys
                            165                 170                 175


        Gln Asp Ser Leu Ser Ser Gln Asn Gln Leu Gly Val Leu Pro Leu Ser
                        180                 185                 190


        Trp Asp Ile Pro Glu Leu Val Asn Met Gly Gln Trp Lys Ile Arg Ala
                        195                 200                 205
```

```
Tyr Tyr Glu Asn Ser Pro Gln Gln Val Phe Ser Thr Glu Phe Glu Val
    210             215             220

Lys Glu Tyr Val Leu Pro Ser Phe Glu Val Ile Val Glu Pro Thr Glu
225             230             235             240

Lys Phe Tyr Tyr Ile Tyr Asn Glu Lys Gly Leu Glu Val Thr Ile Thr
            245             250             255

Ala Arg Phe Leu Tyr Gly Lys Lys Val Glu Gly Thr Ala Phe Val Ile
        260             265             270

Phe Gly Ile Gln Asp Gly Glu Gln Arg Ile Ser Leu Pro Glu Ser Leu
        275             280             285

Lys Arg Ile Pro Ile Glu Asp Gly Ser Gly Glu Val Val Leu Ser Arg
    290             295             300

Lys Val Leu Leu Asp Gly Val Gln Asn Pro Arg Ala Glu Asp Leu Val
305             310             315             320

Gly Lys Ser Leu Tyr Val Ser Ala Thr Val Ile Leu His Ser Gly Ser
            325             330             335

Asp Met Val Gln Ala Glu Arg Ser Gly Ile Pro Ile Val Thr Ser Pro
        340             345             350

Tyr Gln Ile His Phe Thr Lys Thr Pro Lys Tyr Phe Lys Pro Gly Met
        355             360             365

Pro Phe Asp Leu Met Val Phe Val Thr Asn Pro Asp Gly Ser Pro Ala
    370             375             380

Tyr Arg Val Pro Val Ala Val Gln Gly Glu Asp Thr Val Gln Ser Leu
385             390             395             400

Thr Gln Gly Asp Gly Val Ala Lys Leu Ser Ile Asn Thr His Pro Ser
            405             410             415

Gln Lys Pro Leu Ser Ile Thr Val Arg Thr Lys Lys Gln Glu Leu Ser
        420             425             430

Glu Ala Glu Gln Ala Thr Arg Thr Met Gln Ala Leu Pro Tyr Ser Thr
        435             440             445

Val Gly Asn Ser Asn Asn Tyr Leu His Leu Ser Val Leu Arg Thr Glu
    450             455             460
```

46

```
Leu Arg Pro Gly Glu Thr Leu Asn Val Asn Phe Leu Leu Arg Met Asp
465             470             475             480

Arg Ala His Glu Ala Lys Ile Arg Tyr Tyr Thr Tyr Leu Ile Met Asn
                485             490             495

Lys Gly Arg Leu Leu Lys Ala Gly Arg Gln Val Arg Glu Pro Gly Gln
            500             505             510

Asp Leu Val Val Leu Pro Leu Ser Ile Thr Thr Asp Phe Ile Pro Ser
            515             520             525

Phe Arg Leu Val Ala Tyr Tyr Thr Leu Ile Gly Ala Ser Gly Gln Arg
        530             535             540

Glu Val Val Ala Asp Ser Val Trp Val Asp Val Lys Asp Ser Cys Val
545             550             555             560

Gly Ser Leu Val Val Lys Ser Gly Gln Ser Glu Asp Arg Gln Pro Val
            565             570             575

Pro Gly Gln Gln Met Thr Leu Lys Ile Glu Gly Asp His Gly Ala Arg
            580             585             590

Val Val Leu Val Ala Val Asp Lys Gly Val Phe Val Leu Asn Lys Lys
        595             600             605

Asn Lys Leu Thr Gln Ser Lys Ile Trp Asp Val Val Glu Lys Ala Asp
    610             615             620

Ile Gly Cys Thr Pro Gly Ser Gly Lys Asp Tyr Ala Gly Val Phe Ser
625             630             635             640

Asp Ala Gly Leu Thr Phe Thr Ser Ser Gly Gln Gln Thr Ala Gln
            645             650             655

Arg Ala Glu Leu Gln Cys Pro Gln Pro Ala Ala Arg Arg Arg Arg Ser
        660             665             670

Val Gln Leu Thr Glu Lys Arg Met Asp Lys Val Gly Lys Tyr Pro Lys
        675             680             685

Glu Leu Arg Lys Cys Cys Glu Asp Gly Met Arg Glu Asn Pro Met Arg
    690             695             700

Phe Ser Cys Gln Arg Arg Thr Arg Phe Ile Ser Leu Gly Glu Ala Cys
```

705                    710                    715                    720

Lys Lys Val Phe Leu Asp Cys Cys Asn Tyr Ile Thr Glu Leu Arg Arg
            725                 730                 735

Gln His Ala Arg Ala Ser His Leu Gly Leu Ala Arg Ser Asn Leu Asp
            740                 745                 750

Glu Asp Ile Ile Ala Glu Glu Asn Ile Val Ser Arg Ser Glu Phe Pro
            755                 760                 765

Glu Ser Trp Leu Trp Asn Val Glu Asp Leu Lys Glu Pro Pro Lys Asn
    770                 775                 780

Gly Ile Ser Thr Lys Leu Met Asn Ile Phe Leu Lys Asp Ser Ile Thr
785                 790                 795                 800

Thr Trp Glu Ile Leu Ala Val Ser Met Ser Asp Lys Lys Gly Ile Cys
                805                 810                 815

Val Ala Asp Pro Phe Glu Val Thr Val Met Gln Asp Phe Phe Ile Asp
            820                 825                 830

Leu Arg Leu Pro Tyr Ser Val Val Arg Asn Glu Gln Val Glu Ile Arg
            835                 840                 845

Ala Val Leu Tyr Asn Tyr Arg Gln Asn Gln Glu Leu Lys Val Arg Val
    850                 855                 860

Glu Leu Leu His Asn Pro Ala Phe Cys Ser Leu Ala Thr Thr Lys Arg
865                 870                 875                 880

Arg His Gln Gln Thr Val Thr Ile Pro Pro Lys Ser Ser Leu Ser Val
            885                 890                 895

Pro Tyr Val Ile Val Pro Leu Lys Thr Gly Leu Gln Glu Val Glu Val
            900                 905                 910

Lys Ala Ala Val Tyr His His Phe Ile Ser Asp Gly Val Arg Lys Ser
    915                 920                 925

Leu Lys Val Val Pro Glu Gly Ile Arg Met Asn Lys Thr Val Ala Val
    930                 935                 940

Arg Thr Leu Asp Pro Glu Arg Leu Gly Arg Glu Gly Val Gln Lys Glu
945                 950                 955                 960

```
Asp Ile Pro Pro Ala Asp Leu Ser Asp Gln Val Pro Asp Thr Glu Ser
            965                 970                 975


Glu Thr Arg Ile Leu Leu Gln Gly Thr Pro Val Ala Gln Met Thr Glu
            980                 985                 990


Asp Ala Val Asp Ala Glu Arg Leu  Lys His Leu Ile Val  Thr Pro Ser
        995                 1000                 1005


Gly Cys Gly Glu Gln Asn Met  Ile Gly Met Thr Pro  Thr Val Ile
    1010                 1015                 1020


Ala Val His Tyr Leu Asp Glu  Thr Glu Gln Trp Glu  Lys Phe Gly
    1025                 1030                 1035


Leu Glu Lys Arg Gln Gly Ala  Leu Glu Leu Ile Lys  Lys Gly Tyr
    1040                 1045                 1050


Thr Gln Gln Leu Ala Phe Arg  Gln Pro Ser Ser Ala  Phe Ala Ala
    1055                 1060                 1065


Phe Val Lys Arg Ala Pro Ser  Thr Trp Leu Thr Ala  Tyr Val Val
    1070                 1075                 1080


Lys Val Phe Ser Leu Ala Val  Asn Leu Ile Ala Ile  Asp Ser Gln
    1085                 1090                 1095


Val Leu Cys Gly Ala Val Lys  Trp Leu Ile Leu Glu  Lys Gln Lys
    1100                 1105                 1110


Pro Asp Gly Val Phe Gln Glu  Asp Ala Pro Val Ile  His Gln Glu
    1115                 1120                 1125


Met Ile Gly Gly Leu Arg Asn  Asn Asn Glu Lys Asp  Met Ala Leu
    1130                 1135                 1140


Thr Ala Phe Val Leu Ile Ser  Leu Gln Glu Ala Lys  Asp Ile Cys
    1145                 1150                 1155


Glu Glu Gln Val Asn Ser Leu  Pro Gly Ser Ile Thr  Lys Ala Gly
    1160                 1165                 1170


Asp Phe Leu Glu Ala Asn Tyr  Met Asn Leu Gln Arg  Ser Tyr Thr
    1175                 1180                 1185


Val Ala Ile Ala Gly Tyr Ala  Leu Ala Gln Met Gly  Arg Leu Lys
    1190                 1195                 1200
```

```
Gly Pro Leu Leu Asn Lys Phe Leu Thr Thr Ala Lys Asp Lys Asn
    1205             1210             1215

Arg Trp Glu Asp Pro Gly Lys Gln Leu Tyr Asn Val Glu Ala Thr
    1220             1225             1230

Ser Tyr Ala Leu Leu Ala Leu Leu Gln Leu Lys Asp Phe Asp Phe
    1235             1240             1245

Val Pro Pro Val Val Arg Trp Leu Asn Glu Gln Arg Tyr Tyr Gly
    1250             1255             1260

Gly Gly Tyr Gly Ser Thr Gln Ala Thr Phe Met Val Phe Gln Ala
    1265             1270             1275

Leu Ala Gln Tyr Gln Lys Asp Ala Pro Asp His Gln Glu Leu Asn
    1280             1285             1290

Leu Asp Val Ser Leu Gln Leu Pro Ser Arg Ser Ser Lys Ile Thr
    1295             1300             1305

His Arg Ile His Trp Glu Ser Ala Ser Leu Leu Arg Ser Glu Glu
    1310             1315             1320

Thr Lys Glu Asn Glu Gly Phe Thr Val Thr Ala Glu Gly Lys Gly
    1325             1330             1335

Gln Gly Thr Leu Ser Val Val Thr Met Tyr His Ala Lys Ala Lys
    1340             1345             1350

Asp Gln Leu Thr Cys Asn Lys Phe Asp Leu Lys Val Thr Ile Lys
    1355             1360             1365

Pro Ala Pro Glu Thr Glu Lys Arg Pro Gln Asp Ala Lys Asn Thr
    1370             1375             1380

Met Ile Leu Glu Ile Cys Thr Arg Tyr Arg Gly Asp Gln Asp Ala
    1385             1390             1395

Thr Met Ser Ile Leu Asp Ile Ser Met Met Thr Gly Phe Ala Pro
    1400             1405             1410

Asp Thr Asp Asp Leu Lys Gln Leu Ala Asn Gly Val Asp Arg Tyr
    1415             1420             1425

Ile Ser Lys Tyr Glu Leu Asp Lys Ala Phe Ser Asp Arg Asn Thr
    1430             1435             1440
```

```
Leu Ile Ile Tyr Leu Asp Lys Val Ser His Ser Glu Asp Asp Cys
    1445              1450              1455

Leu Ala Phe Lys Val His Gln Tyr Phe Asn Val Glu Leu Ile Gln
    1460              1465              1470

Pro Gly Ala Val Lys Val Tyr Ala Tyr Tyr Asn Leu Glu Glu Ser
    1475              1480              1485

Cys Thr Arg Phe Tyr His Pro Glu Lys Glu Asp Gly Lys Leu Asn
    1490              1495              1500

Lys Leu Cys Arg Asp Glu Leu Cys Arg Cys Ala Glu Glu Asn Cys
    1505              1510              1515

Phe Ile Gln Lys Ser Asp Asp Lys Val Thr Leu Glu Glu Arg Leu
    1520              1525              1530

Asp Lys Ala Cys Glu Pro Gly Val Asp Tyr Val Tyr Lys Thr Arg
    1535              1540              1545

Leu Val Lys Val Gln Leu Ser Asn Asp Phe Asp Glu Tyr Ile Met
    1550              1555              1560

Ala Ile Glu Gln Thr Ile Lys Ser Gly Ser Asp Glu Val Gln Val
    1565              1570              1575

Gly Gln Gln Arg Thr Phe Ile Ser Pro Ile Lys Cys Arg Glu Ala
    1580              1585              1590

Leu Lys Leu Glu Glu Lys Lys His Tyr Leu Met Trp Gly Leu Ser
    1595              1600              1605

Ser Asp Phe Trp Gly Glu Lys Pro Asn Leu Ser Tyr Ile Ile Gly
    1610              1615              1620

Lys Asp Thr Trp Val Glu His Trp Pro Glu Glu Asp Glu Cys Gln
    1625              1630              1635

Asp Glu Glu Asn Gln Lys Gln Cys Gln Asp Leu Gly Ala Phe Thr
    1640              1645              1650

Glu Ser Met Val Val Phe Gly Cys Pro Asn
    1655              1660
```

<210> 40
<211> 866

<212> PRT
<213> Homo sapiens

<400> 40

Met Phe Ser Met Arg Ile Val Cys Leu Val Leu Ser Val Val Gly Thr
1           5              10             15

Ala Trp Thr Ala Asp Ser Gly Glu Gly Asp Phe Leu Ala Glu Gly Gly
        20          25                30

Gly Val Arg Gly Pro Arg Val Val Glu Arg His Gln Ser Ala Cys Lys
        35          40                45

Asp Ser Asp Trp Pro Phe Cys Ser Asp Glu Asp Trp Asn Tyr Lys Cys
    50          55              60

Pro Ser Gly Cys Arg Met Lys Gly Leu Ile Asp Glu Val Asn Gln Asp
65              70              75              80

Phe Thr Asn Arg Ile Asn Lys Leu Lys Asn Ser Leu Phe Glu Tyr Gln
            85              90              95

Lys Asn Asn Lys Asp Ser His Ser Leu Thr Thr Asn Ile Met Glu Ile
        100             105             110

Leu Arg Gly Asp Phe Ser Ser Ala Asn Asn Arg Asp Asn Thr Tyr Asn
        115             120             125

Arg Val Ser Glu Asp Leu Arg Ser Arg Ile Glu Val Leu Lys Arg Lys
    130             135             140

Val Ile Glu Lys Val Gln His Ile Gln Leu Leu Gln Lys Asn Val Arg
145             150             155             160

Ala Gln Leu Val Asp Met Lys Arg Leu Glu Val Asp Ile Asp Ile Lys
            165             170             175

Ile Arg Ser Cys Arg Gly Ser Cys Ser Arg Ala Leu Ala Arg Glu Val
        180             185             190

Asp Leu Lys Asp Tyr Glu Asp Gln Gln Lys Gln Leu Glu Gln Val Ile
        195             200             205

Ala Lys Asp Leu Leu Pro Ser Arg Asp Arg Gln His Leu Pro Leu Ile
    210             215             220

Lys Met Lys Pro Val Pro Asp Leu Val Pro Gly Asn Phe Lys Ser Gln

53

225            230            235            240

Leu Gln Lys Val Pro Pro Glu Trp Lys Ala Leu Thr Asp Met Pro Gln
               245             250             255

Met Arg Met Glu Leu Glu Arg Pro Gly Gly Asn Glu Ile Thr Arg Gly
         260             265             270

Gly Ser Thr Ser Tyr Gly Thr Gly Ser Glu Thr Glu Ser Pro Arg Asn
         275             280             285

Pro Ser Ser Ala Gly Ser Trp Asn Ser Gly Ser Ser Gly Pro Gly Ser
         290             295             300

Thr Gly Asn Arg Asn Pro Gly Ser Ser Gly Thr Gly Gly Thr Ala Thr
305             310             315             320

Trp Lys Pro Gly Ser Ser Gly Pro Gly Ser Thr Gly Ser Trp Asn Ser
         325             330             335

Gly Ser Ser Gly Thr Gly Ser Thr Gly Asn Gln Asn Pro Gly Ser Pro
         340             345             350

Arg Pro Gly Ser Thr Gly Thr Trp Asn Pro Gly Ser Ser Glu Arg Gly
         355             360             365

Ser Ala Gly His Trp Thr Ser Glu Ser Ser Val Ser Gly Ser Thr Gly
         370             375             380

Gln Trp His Ser Glu Ser Gly Ser Phe Arg Pro Asp Ser Pro Gly Ser
385             390             395             400

Gly Asn Ala Arg Pro Asn Asn Pro Asp Trp Gly Thr Phe Glu Glu Val
         405             410             415

Ser Gly Asn Val Ser Pro Gly Thr Arg Arg Glu Tyr His Thr Glu Lys
         420             425             430

Leu Val Thr Ser Lys Gly Asp Lys Glu Leu Arg Thr Gly Lys Glu Lys
         435             440             445

Val Thr Ser Gly Ser Thr Thr Thr Thr Arg Arg Ser Cys Ser Lys Thr
         450             455             460

Val Thr Lys Thr Val Ile Gly Pro Asp Gly His Lys Glu Val Thr Lys
465             470             475             480

```
Glu Val Val Thr Ser Glu Asp Gly Ser Asp Cys Pro Glu Ala Met Asp
                485             490                 495

Leu Gly Thr Leu Ser Gly Ile Gly Thr Leu Asp Gly Phe Arg His Arg
                500             505                 510

His Pro Asp Glu Ala Ala Phe Phe Asp Thr Ala Ser Thr Gly Lys Thr
                515             520                 525

Phe Pro Gly Phe Phe Ser Pro Met Leu Gly Glu Phe Val Ser Glu Thr
                530             535                 540

Glu Ser Arg Gly Ser Glu Ser Gly Ile Phe Thr Asn Thr Lys Glu Ser
545             550             555                 560

Ser Ser His His Pro Gly Ile Ala Glu Phe Pro Ser Arg Gly Lys Ser
                565             570                 575

Ser Ser Tyr Ser Lys Gln Phe Thr Ser Ser Thr Ser Tyr Asn Arg Gly
                580             585                 590

Asp Ser Thr Phe Glu Ser Lys Ser Tyr Lys Met Ala Asp Glu Ala Gly
                595             600                 605

Ser Glu Ala Asp His Glu Gly Thr His Ser Thr Lys Arg Gly His Ala
                610             615                 620

Lys Ser Arg Pro Val Arg Asp Cys Asp Asp Val Leu Gln Thr His Pro
625             630             635                 640

Ser Gly Thr Gln Ser Gly Ile Phe Asn Ile Lys Leu Pro Gly Ser Ser
                645             650                 655

Lys Ile Phe Ser Val Tyr Cys Asp Gln Glu Thr Ser Leu Gly Gly Trp
                660             665                 670

Leu Leu Ile Gln Gln Arg Met Asp Gly Ser Leu Asn Phe Asn Arg Thr
                675             680                 685

Trp Gln Asp Tyr Lys Arg Gly Phe Gly Ser Leu Asn Asp Glu Gly Glu
                690             695                 700

Gly Glu Phe Trp Leu Gly Asn Asp Tyr Leu His Leu Leu Thr Gln Arg
705             710             715                 720

Gly Ser Val Leu Arg Val Glu Leu Glu Asp Trp Ala Gly Asn Glu Ala
                725             730                 735
```

55

```
Tyr Ala Glu Tyr His Phe Arg Val Gly Ser Glu Ala Glu Gly Tyr Ala
            740                 745             750

Leu Gln Val Ser Ser Tyr Glu Gly Thr Ala Gly Asp Ala Leu Ile Glu
            755                 760             765

Gly Ser Val Glu Glu Gly Ala Glu Tyr Thr Ser His Asn Asn Met Gln
            770                 775             780

Phe Ser Thr Phe Asp Arg Asp Ala Asp Gln Trp Glu Glu Asn Cys Ala
785                 790                 795                 800

Glu Val Tyr Gly Gly Gly Trp Trp Tyr Asn Asn Cys Gln Ala Ala Asn
                805                 810                 815

Leu Asn Gly Ile Tyr Tyr Pro Gly Gly Ser Tyr Asp Pro Arg Asn Asn
            820                 825                 830

Ser Pro Tyr Glu Ile Glu Asn Gly Val Val Trp Val Ser Phe Arg Gly
            835                 840                 845

Ala Asp Tyr Ser Leu Arg Ala Val Arg Met Lys Ile Arg Pro Leu Val
    850                 855                 860

Thr Gln
865
```

<210> 41
<211> 418
<212> PRT
<213> Homo sapiens

<400> 41

```
Met Pro Ser Ser Val Ser Trp Gly Ile Leu Leu Leu Ala Gly Leu Cys
1               5               10                  15

Cys Leu Val Pro Val Ser Leu Ala Glu Asp Pro Gln Gly Asp Ala Ala
            20                  25                  30

Gln Lys Thr Asp Thr Ser His His Asp Gln Asp His Pro Thr Phe Asn
            35                  40                  45

Lys Ile Thr Pro Asn Leu Ala Glu Phe Ala Phe Ser Leu Tyr Arg Gln
        50                  55                  60

Leu Ala His Gln Ser Asn Ser Thr Asn Ile Phe Phe Ser Pro Val Ser
65                  70                  75                  80
```

```
Ile Ala Thr Ala Phe Ala Met Leu Ser Leu Gly Thr Lys Ala Asp Thr
            85              90                  95

His Asp Glu Ile Leu Glu Gly Leu Asn Phe Asn Leu Thr Glu Ile Pro
            100             105                 110

Glu Ala Gln Ile His Glu Gly Phe Gln Glu Leu Leu Arg Thr Leu Asn
            115             120                 125

Gln Pro Asp Ser Gln Leu Gln Leu Thr Thr Gly Asn Gly Leu Phe Leu
    130             135             140

Ser Glu Gly Leu Lys Leu Val Asp Lys Phe Leu Glu Asp Val Lys Lys
145             150             155             160

Leu Tyr His Ser Glu Ala Phe Thr Val Asn Phe Gly Asp Thr Glu Glu
            165             170                 175

Ala Lys Lys Gln Ile Asn Asp Tyr Val Glu Lys Gly Thr Gln Gly Lys
            180             185                 190

Ile Val Asp Leu Val Lys Glu Leu Asp Arg Asp Thr Val Phe Ala Leu
            195             200                 205

Val Asn Tyr Ile Phe Phe Lys Gly Lys Trp Glu Arg Pro Phe Glu Val
    210             215             220

Lys Asp Thr Glu Glu Glu Asp Phe His Val Asp Gln Val Thr Thr Val
225             230             235             240

Lys Val Pro Met Met Lys Arg Leu Gly Met Phe Asn Ile Gln His Cys
            245             250                 255

Lys Lys Leu Ser Ser Trp Val Leu Leu Met Lys Tyr Leu Gly Asn Ala
            260             265                 270

Thr Ala Ile Phe Phe Leu Pro Asp Glu Gly Lys Leu Gln His Leu Glu
            275             280                 285

Asn Glu Leu Thr His Asp Ile Ile Thr Lys Phe Leu Glu Asn Glu Asp
    290             295             300

Arg Arg Ser Ala Ser Leu His Leu Pro Lys Leu Ser Ile Thr Gly Thr
305             310             315             320

Tyr Asp Leu Lys Ser Val Leu Gly Gln Leu Gly Ile Thr Lys Val Phe
            325             330                 335
```

```
Ser Asn Gly Ala Asp Leu Ser Gly Val Thr Glu Glu Ala Pro Leu Lys
            340             345             350

Leu Ser Lys Ala Val His Lys Ala Val Leu Thr Ile Asp Glu Lys Gly
            355             360             365

Thr Glu Ala Ala Gly Ala Met Phe Leu Glu Ala Ile Pro Met Ser Ile
            370             375             380

Pro Pro Glu Val Lys Phe Asn Lys Pro Phe Val Phe Leu Met Ile Glu
385             390             395             400

Gln Asn Thr Lys Ser Pro Leu Phe Met Gly Lys Val Val Asn Pro Thr
            405             410             415

Gln Lys
```

<210> 42
<211> 453
<212> PRT
<213> Homo sapiens

<400> 42

```
Met Ser Trp Ser Leu His Pro Arg Asn Leu Ile Leu Tyr Phe Tyr Ala
1               5               10              15

Leu Leu Phe Leu Ser Ser Thr Cys Val Ala Tyr Val Ala Thr Arg Asp
            20              25              30

Asn Cys Cys Ile Leu Asp Glu Arg Phe Gly Ser Tyr Cys Pro Thr Thr
            35              40              45

Cys Gly Ile Ala Asp Phe Leu Ser Thr Tyr Gln Thr Lys Val Asp Lys
            50              55              60

Asp Leu Gln Ser Leu Glu Asp Ile Leu His Gln Val Glu Asn Lys Thr
65              70              75              80

Ser Glu Val Lys Gln Leu Ile Lys Ala Ile Gln Leu Thr Tyr Asn Pro
            85              90              95

Asp Glu Ser Ser Lys Pro Asn Met Ile Asp Ala Ala Thr Leu Lys Ser
            100             105             110

Arg Lys Met Leu Glu Glu Ile Met Lys Tyr Glu Ala Ser Ile Leu Thr
            115             120             125
```

```
His Asp Ser Ser Ile Arg Tyr Leu Gln Glu Ile Tyr Asn Ser Asn Asn
    130                 135                 140

Gln Lys Ile Val Asn Leu Lys Glu Lys Val Ala Gln Leu Glu Ala Gln
    145                 150                 155                 160

Cys Gln Glu Pro Cys Lys Asp Thr Val Gln Ile His Asp Ile Thr Gly
                165                 170                 175

Lys Asp Cys Gln Asp Ile Ala Asn Lys Gly Ala Lys Gln Ser Gly Leu
                180                 185                 190

Tyr Phe Ile Lys Pro Leu Lys Ala Asn Gln Gln Phe Leu Val Tyr Cys
            195                 200                 205

Glu Ile Asp Gly Ser Gly Asn Gly Trp Thr Val Phe Gln Lys Arg Leu
    210                 215                 220

Asp Gly Ser Val Asp Phe Lys Lys Asn Trp Ile Gln Tyr Lys Glu Gly
225                 230                 235                 240

Phe Gly His Leu Ser Pro Thr Gly Thr Thr Glu Phe Trp Leu Gly Asn
                245                 250                 255

Glu Lys Ile His Leu Ile Ser Thr Gln Ser Ala Ile Pro Tyr Ala Leu
        260                 265                 270

Arg Val Glu Leu Glu Asp Trp Asn Gly Arg Thr Ser Thr Ala Asp Tyr
        275                 280                 285

Ala Met Phe Lys Val Gly Pro Glu Ala Asp Lys Tyr Arg Leu Thr Tyr
    290                 295                 300

Ala Tyr Phe Ala Gly Gly Asp Ala Gly Asp Ala Phe Asp Gly Phe Asp
305                 310                 315                 320

Phe Gly Asp Asp Pro Ser Asp Lys Phe Phe Thr Ser His Asn Gly Met
                325                 330                 335

Gln Phe Ser Thr Trp Asp Asn Asp Asn Asp Lys Phe Glu Gly Asn Cys
            340                 345                 350

Ala Glu Gln Asp Gly Ser Gly Trp Trp Met Asn Lys Cys His Ala Gly
        355                 360                 365

His Leu Asn Gly Val Tyr Tyr Gln Gly Gly Thr Tyr Ser Lys Ala Ser
```

59

<pre>
        370                   375                       380

Thr Pro Asn Gly Tyr Asp Asn Gly Ile Ile Trp Ala Thr Trp Lys Thr
385                 390             395                     400


Arg Trp Tyr Ser Met Lys Lys Thr Thr Met Lys Ile Ile Pro Phe Asn
            405             410             415


Arg Leu Thr Ile Gly Glu Gly Gln Gln His His Leu Gly Gly Ala Lys
            420             425             430


Gln Val Arg Pro Glu His Pro Ala Glu Thr Glu Tyr Asp Ser Leu Tyr
            435             440             445


Pro Glu Asp Asp Leu
        450
</pre>

<210> 43
<211> 330
<212> PRT
<213> Homo sapiens

<400> 43

Ala Ser Thr Lys Gly Pro Ser Val Phe Pro Leu Ala Pro Ser Ser Lys
1                   5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
            115                 120                 125

Lys Pro Lys Asp Thr Leu Met Ile Ser Arg Thr Pro Glu Val Thr Cys

                    130                         135                              140

Val Val Val Asp Val Ser His Glu Asp Pro Glu Val Lys Phe Asn Trp
145                   150                   155                   160

Tyr Val Asp Gly Val Glu Val His Asn Ala Lys Thr Lys Pro Arg Glu
                  165                   170                   175

Glu Gln Tyr Asn Ser Thr Tyr Arg Val Val Ser Val Leu Thr Val Leu
                  180                   185                   190

His Gln Asp Trp Leu Asn Gly Lys Glu Tyr Lys Cys Lys Val Ser Asn
                  195                   200                   205

Lys Ala Leu Pro Ala Pro Ile Glu Lys Thr Ile Ser Lys Ala Lys Gly
          210                   215                   220

Gln Pro Arg Glu Pro Gln Val Tyr Thr Leu Pro Pro Ser Arg Asp Glu
225                   230                   235                   240

Leu Thr Lys Asn Gln Val Ser Leu Thr Cys Leu Val Lys Gly Phe Tyr
                  245                   250                   255

Pro Ser Asp Ile Ala Val Glu Trp Glu Ser Asn Gly Gln Pro Glu Asn
          260                   265                   270

Asn Tyr Lys Thr Thr Pro Pro Val Leu Asp Ser Asp Gly Ser Phe Phe
          275                   280                   285

Leu Tyr Ser Lys Leu Thr Val Asp Lys Ser Arg Trp Gln Gln Gly Asn
    290                   295                   300

Val Phe Ser Cys Ser Val Met His Glu Ala Leu His Asn His Tyr Thr
305                   310                   315                   320

Gln Lys Ser Leu Ser Leu Ser Pro Gly Lys
                  325                   330

<210> 44
<211> 15
<212> PRT
<213> Homo sapiens

<220>
<221> MISC_FEATURE
<222> (3)..(3)
<223> methionine may be oxidized

<220>

<221> MISC_FEATURE
<222> (12)..(12)
<223> glycated

<220>
<221> MISC_FEATURE
<222> (13)..(13)
<223> Cysteine may be alkylated

<400> 44

```
        Ala Val Met Asp Asp Phe Ala Ala Phe Val Glu Lys Cys Cys Lys
        1                   5                   10                  15
```

<210> 45
<211> 23
<212> PRT
<213> Homo sapiens

<220>
<221> MISC_FEATURE
<222> (10)..(10)
<223> Glycated

<220>
<221> MISC_FEATURE
<222> (12)..(12)
<223> Cysteine may be alkylated

<220>
<221> MISC_FEATURE
<222> (21)..(21)
<223> Cysteine may be alkylated

<400> 45

```
        Leu Val Asn Glu Val Thr Glu Phe Ala Lys Thr Cys Val Ala Asp Glu
        1                   5                   10                  15

        Ser Ala Glu Asn Cys Asp Lys
                    20
```

<210> 46
<211> 15
<212> PRT
<213> Homo sapiens

<220>
<221> MISC_FEATURE
<222> (3)..(3)
<223> Methionine may be oxidized

<220>
<221> MISC_FEATURE
<222> (12)..(12)
<223> glycated

```
<220>
<221> MISC_FEATURE
<222> (13)..(14)
<223> Cysteine may be alkylated

<400> 46

        Ala Val Met Asp Asp Phe Ala Ala Phe Val Glu Lys Cys Cys Lys
        1               5                   10                  15
```

**Beschreibung**

Die Erfindung betrifft ein Verfahren und Mittel zur nichtinvasiven Diagnostik von Typ-II-Diabetes mellitus.

Typ-II-Diabetes mellitus ist eine Störung, bei der Insulin zwar vorhanden ist, an seinem Zielort, den Zellmembranen, aber nicht richtig wirken kann (Insulinresistenz). In den ersten Krankheitsjahren kann die Bauchspeicheldrüse dies durch die Produktion hoher Insulinmengen kompensieren. Irgendwann kann die Bauchspeicheldrüse die überhöhte Insulinproduktion aber nicht mehr aufrechterhalten. Die produzierte Insulinmenge reicht dann nicht mehr aus, um den Blutzuckerspiegel zu kontrollieren, und der Diabetes mellitus Typ 2 wird manifest. Aufgrund einer hohen Insulinresistenz steigt der Blutzucker dennoch an, später kommt es über einen relativen Mangel in einigen Fällen zu einem absoluten Insulinmangel.

Im Gegensatz zum Typ-1-Diabetes geht der Typ-2-Diabetes eher selten mit einer Gewichtsabnahme und nur bei massiv erhöhten Blutzuckerwerten mit vermehrtem Wasserlassen und Durstgefühl einher. Häufig bestehen zu Beginn unspezifische Symptome wie Müdigkeit, Schwäche, Sehstörungen und Infektneigung wie z. B. häufige Blasenentzündungen. Da diese Symptome sehr unspezifisch sind, wird die Diagnose häufig erst nach Jahren durch Zufall gestellt. Zu diesem Zeitpunkt können jedoch bereits zum Teil irreversible gesundheitliche Schädigungen stattgefunden haben.

Daher ist eine möglichst frühzeitige Diagnose wesentlich, um durch geeignete Therapiemaßnahmen eine Manifestierung des Typ-II-Diabetes zu verhindern.

Durch den erhöhten Blutzuckerspiegel kommt es zu einer nicht enzymatischen Reaktion von Zuckern mit Lipiden und Proteinen und der Bildung von Amadori-Produkten durch Amadori-Umlagerung (Glykierung). Endogene Glykierung entsteht auch im Körper, besonders im Blutkreislauf. Hierbei reagieren im Wesentlichen Glukose, Fruktose und Galaktose unkontrolliert mit körpereigenen Proteinen ohne Beteiligung von Enzymen. Problematisch ist hierbei die Akkumulation dieses Effektes über die Zeit, insbesondere bei einem erhöhten Blutzuckerwert, bei der Gewebe oder Zellen geschädigt werden können. Die Glykierung von $HbA_{1c}$ wird daher genutzt um bei Diabetikern eine Langzeitkontrolle von Blutzuckerwerten vorzunehmen. $HbA_{1c}$, auch Glykohämoglobin (GHb) genannt, ist roter Blutfarbstoff (Hämoglobin), der durch Glukose chemisch verändert ist.

Die DE 69835268 T2 offenbart ein Verfahren zum Messen eines glykierten Proteins bereits, indem Protease und FAOD auf das in einer Probe enthaltene glykierte Protein einwirken, wobei eine Protease der Gattung Aspergillus verwendet wird. Dabei kann ein glykiertes Protein in einer Komponente eines lebenden Organismus mit hoher Empfindlichkeit und Genauigkeit gemessen werden durch Verwenden einer geeigneten Protease, die eine verwendbare enzymatische Wirkung in Kombination mit einer FAOD aufweist, die in geeigneter Weise zum Messen von glykiertem Albumin verwendbar ist. Jedoch wird hierbei nur die allgemeine Glykierung des Albumins festgestellt, jedoch keine spezifische Glykierung für die Diagnostik von Typ-II-Diabetes mellitus.

Die EP 0 623 216 B1 offenbart einen Antikörper der spezifisch auf glykierte Proteine reagiert, wobei als solches Protein auch Humanes Serum Albumin genannt wird.

Die EP 0 230 934 A2 offenbart ein Verfahren bei dem glykierte Lysinreste als Epitop für Antikörper dienen. Dadurch können glykierte Proteine, wie etwa Humanes Serum Albumin, festgestellt werden.

Die WO 2013/159025 A1 offenbart ein Verfahren zur Diagnose von Diabetes, wobei das N-Glykierungsmuster verschiedener Plasmaproteine untersucht wird und Änderungen dieses Musters zur Diagnose verwendet werden.

Zudem offenbart die US 2004/0147033 A1 die Verwendung von Glykoproteine zur Diagnose verschiedener Krankheiten.

Das Dokument WO 2007/117794 A2 offenbart ein Verfahren zur Überwachung des Blutglucose-Spiegels und des glykämischen Zustands von Patienten durch Bestimmung der Konzentration glykierter Peptide im Blut. Dabei werden Antikörper oder -fragmente gegen ausgewählte glykierte Peptide eingesetzt, wobei gemäß der Tabelle 1 die Seq. 21 (DC) ein glykiertes Peptid von Haptoglobin (KQLVEIEK) mit zwei Lysinresten offenbart.

Das Dokument US 2011/0136160 A1 offenbart die Analyse von Glykierungsstellen von humanen Plasmaproteinen. Zur Simulation eines hohen Glukosespiegels entsprechend glukotoxischen Zuständen wurden Proben mit einer Glukosekonzentration von 30 mM inkubiert und danach die Glykierung gängiger Plasmaproteine untersucht. Haptoglobin wurde als glykiertes Protein identifiziert und die Positionen K270 und K151 als glykierte Positionen des Haptoglobin ermittelt.

Die vorbeschriebenen Diagnoseverfahren sind zwar zur Detektion glykierter Proteine, wie etwa Humanem Serum Albumin, geeignet, lassen eine sichere Diagnose von Typ-II-Diabetes jedoch nicht zu.

Die Aufgabe der vorliegenden Erfindung ist daher ein Verfahren zur nichtinvasiven Diagnostik von Typ-II-diabetes mellitus anzugeben, das eine sichere und einfache Diagnose bereits im Frühstadium ermöglicht.

Die Aufgabe wird durch ein Verfahren gemäß Anspruch 1 gelöst. Vorteilhafte Ausgestaltungen sind in den abhängigen Ansprüchen angegeben.

Erfindungsgemäß wird ein Verfahren zur nichtinvasiven Diagnostik von Diabetes, insbesondere Typ-II-Diabetes mellitus, angegeben, wobei zumindest die Bestimmung der Glykierung in der Glykierungsposition Lys 141 von Humanem Haptoglobin (P00738; SEQ ID No. 38) in einer isolierten Blut- oder Plasmaprobe erfolgt,

sowie eine Bestimmung des HbA$_{1c}$-Spiegels erfolgt, wobei nachfolgend die Glykierung in den Glykierungspositionen mit dem HbA$_{1c}$-Spiegel korreliert wird.

Die P-Nummer hinter dem Proteinnamen ist der UniProt Identifier (Accession number). Die Sequenzpositionen beziehen sich auf die Sequenzen die jeweils im beiliegenden Sequenzprotokoll unter der in der Klammer genannten SEQ ID hinterlegt sind. Die Position in humanem Serum Albumin bezieht sich dabei auf das reife Protein ohne das im UniProt-Eintrag angegebene Signalpeptid und ohne Propeptid.

HbA$_{1c}$ wird dabei als das stabile Produkt einer Kopplung von Glukose an das N-terminale Valin der beta-Kette des Hämoglobins A1 definiert (International Federation of Clinical Chemistry and Laboratory Medicine). Gebräuchlich ist nach wie vor eine Angabe in Prozent (%). Die nach Empfehlung der IFCC eingeführte internationale Einheit ist mmol/mol Hämoglobin, also ein Promille-Wert. Zur besseren Unterscheidung von der %-Angabe kann dieser Wert auch als HbA$_{1c}$M bezeichnet werden. Die Umrechnungsformel lautet:

$$HbA_{1c} \text{ [mmol/mol Hb]} = (HbA_{1c}[\%] - 2,15) \times 10,929.$$

Das HbA$_{1c}$ wird aus Vollblut mittels eines Enzymimmunoassays bestimmt.

Für die Umrechnung des durchschnittlichen Blutzuckerwertes in den HbA$_{1c}$-Wert werden folgende Formeln verwendet:

$$HbA_{1c} \text{ [\%]} = (\text{Mittlerer Blutzucker [mg/dl]} + 86) / 33,3$$

$$HbA_{1c} \text{ [\%]} = (\text{Mittlerer Blutzucker(Plasma) [mg/dl]} + 77,3) / 35,6$$

Bevorzugt erfolgt die Bestimmung der Glykierung von humanen Plasmaproteinen in zumindest einer weiteren Glykierungsposition ausgewählt aus

- Lys 174 in Humanem Serum Albumin (P02768, SEQ ID No. 31),
- Lys 414 in Humanem Serum Albumin (P02768, SEQ ID No. 31),
- Lys 574 in Humanem Serum Albumin (P02768, SEQ ID No. 31),
- Lys 163 in der Humanen Fibrinogen beta-Kette (P02675, SEQ ID No. 33),
- Lys 50 in der Humanen Ig Lambda Kette-C-Region (P01842; SEQ ID No. 36),

Bevorzugt wird die Glykierung in mindestens einer Sequenz ausgewählt aus den SEQ ID-No. 1 bis 30 bestimmt. Bevorzugt erfolgt die Bestimmung der Glykierung in zwei bis fünf, besonders bevorzugt drei bis fünf, weiter bevorzugt in allen der SEQ ID-No. 1 bis 30, wobei zumindest eine Bestimmung der Glykierung in der SEQ ID No. 27 erfolgt.

Die Sequenzen der erfindungsgemäßen Glykierungspositionen der humanen Plasmaproteine sind der nachfolgenden Tabelle 1 wiedergegeben:

Tabelle 1

| SEQ ID No. | Sequenz | Protein | Glykierungsposition |
|---|---|---|---|
| 1 | TC*VADESAENC*DK*SLHTLFGDK | Humanes Serum Albumin (P02768, SEQ ID No. 31) | K64 |
| 2 | SLHTLFGDK*LC*TVATLR | Humanes Serum Albumin (P02768, SEQ ID No. 31) | K73 |
| 3 | ETYGEMADC*C*AK*QEPER | Humanes Serum Albumin (P02768, SEQ ID No. 31) | K93 |
| 4 | ETYGEM$_{ox}$ADC*C*AK*QEPER | Humanes Serum Albumin (P02768, SEQ ID No. 31) | K93 |
| *5 | AAFTEC*C*QAADK*AAC*LLPK | Humanes Serum Albumin (P02768, SEQ ID No. 31) | K174 |

(fortgesetzt)

| SEQ ID No. | Sequenz | Protein | Glykierungsposition |
|---|---|---|---|
| 6 | AAC*LLPK*LDELRDEGK | Humanes Serum Albumin (P02768, SEQ ID No. 31) | K181 |
| 7 | AEFAEVSK*LVTDLTK | Humanes Serum Albumin (P02768, SEQ ID No. 31) | K233 |
| 8 | ADLAK*YIC*ENQDSISSK | Humanes Serum Albumin (P02768, SEQ ID No. 31) | K262 |
| 9 | TYETTLEK*C*C*AAADPHEC*YAK | Humanes Serum Albumin (P02768, SEQ ID No. 31) | K359 |
| 10 | VFDEFK*PLVEEPQNLIK | Humanes Serum Albumin (P02768, SEQ ID No. 31) | K378 |
| 11 | K*VPQVSTPTL VEVSR | Humanes Serum Albumin (P02768, SEQ ID No. 31) | K414 |
| 12 | K*QTALVELVK | Humanes Serum Albumin (P02768, SEQ ID No. 31) | K525 |
| 13 | EQLK*AVMDDFAAFVEK | Humanes Serum Albumin (P02768, SEQ ID No. 31) | K545 |
| 14 | K*LVAASQAALGL | Humanes Serum Albumin (P02768, SEQ ID No. 31) | K574 |
| 15 | VQWK*VDNALQSGNSQESVTEQDSK | Humanes Ig Kappa Kette-C-Region (P01834, SEQ ID No. 32) | K41 |
| 16 | DSTYSLSSTLTLSK*ADYEK | Humanes Ig Kappa Kette-C-Region (P01834, SEQ ID No. 32) | K75 |
| 17 | VYAC*EVTHQGLSSPVTK*SFNR | Humanes Ig Kappa Kette-C-Region (P01834, SEQ ID No. 32) | K99 |
| 18 | QVK*DNENVVNEYSSELEK | Humane Fibrinogen beta-Kette (P02675, SEQ ID No. 33) | K163 |
| 19 | IQK*LESDVSAQM$_{ox}$EYC*R | Humane Fibrinogen beta-Kette (P02675, SEQ ID No. 33) | K211 |
| 20 | K*WDPYKQGFGNVATNTDGK | Humane Fibrinogen beta-Kette (P02675, SEQ ID No. 33) | K295 |
| 21 | SK*AIGYLNTGYQR | Humanes Alpha-2-macroglobulin (P01023, SEQ ID No. 34) | K1003 |
| 22 | ALLAYAFALAGNQDK*R | Humanes Alpha-2-macroglobulin (P01023, SEQ ID No. 34) | K1162 |
| 23 | K*C*STSSLLEAC*TFR | Humanes Serotransferrin (P02787; SEQ ID No. 35) | K683 |
| 24 | ADSSPVK*AGVETTTPSK | Humane Ig Lambda Kette-C-Region P01842; SEQ ID No. 36) | K50 |
| 25 | AKvVQPYLDDFQK | Humanes Apolipoprotein A-I precursor (P02647; SEQ ID No. 37) | K120 |
| 26 | K*WQEEM$_{ox}$ELYR | Humanes Apolipoprotein A-I precursor (P02647; SEQ ID No. 37 | K131 |

(fortgesetzt)

| SEQ ID No. | Sequenz | Protein | Glykierungsposition |
|---|---|---|---|
| 27 | AVGDK*LPEC*EAVC*GKPK | Humanes Haptoglobin (P00738; SEQ ID No. 38) | K141 |
| 28 | SEETK*ENEGFTVTAEGK | Humaner Complement C3 precursor (P01024; SEQ ID No. 39) | K1325 |
| 29 | M$_{ox}$K*GLIDEVNQDFTNR | Humane Fibrinogen alpha-Kette (P02671; SEQ ID No. 40) | K71 |
| 30 | SSSYSK*QFTSSTSYNR | Humane Fibrinogen alpha-Kette (P02671; SEQ ID No. 40) | K581 |

Die Sequenzpositionen beziehen sich auf die Sequenzen, die jeweils im beiliegenden Sequenzprotokoll unter der in der Klammer genannten SEQ ID hinterlegt sind. Die Positionen im Humanen Serum Albumin beziehen sich dabei auf das reife Protein ohne das im UniProt-Eintrag angegebene Signalpeptid und ohne Propeptid.

Die Glykierung erfolgt dabei an der in der Tab. 1 angegebenen Position der Lysinreste (K) der Plasmaproteine. Bevorzugt wird durch Glykierung am Lysin (K) jeweils ein Amadori Produkt gebildet. Die Sequenzen können zusätzlich an einem oder mehreren Cysteinen (C) in Folge des verwendeten Protokolls zur enzymatischen Spaltung der Plasma- oder Serumprobe alkyliert sein. Optional ist der Schwefel in den Methioninen jeweils (zum Sulfoxid) oxidiert. Dabei bedeuten K*=Fruktosamin-modifiziertes Lysin, C*=Carbamidomethyliertes Cystein und M$_{Ox}$ = Methioninsulfoxid.

Bevorzugt erfolgt die Bestimmung der Glykierung an zwei bis fünfzehn, besonders bevorzugt fünf bis zehn der oben genannten Glykierungspositionen wobei zumindest eine Bestimmung der Glykierung in der SEQ ID No. 27 erfolgt.

In einer Ausführungsform der Erfindung erfolgt im hyperglykämischen Zustand bei Typ-II-Diabetes-Patienten eine spezifische Glykierung der Lysinreste

-   Lys 174 in Humanem Serum Albumin (P02768, SEQ ID No. 31),

-   Lys 414 in Humanem Serum Albumin (P02768, SEQ ID No. 31),

-   Lys 574 in Humanem Serum Albumin (P02768, SEQ ID No. 31),

-   Lys 163 in der Humanen Fibrinogen beta-Kette (P02675, SEQ ID No. 33),

-   Lys 50 in der Humanen Ig Lambda Kette-C-Region (P01842; SEQ ID No. 36),

-   Lys 141 von Humanem Haptoglobin (P00738; SEQ ID No. 38).

bzw. in den Glykierungspositionen der Sequenzen gemäß SEQ ID No. 5, 11, 14, 18, 24 und 27.Im Vergleich dazu konnte in einer Kontrollgruppe keinerlei Glykierung in den Glykierungspositionen der oben genannten Lysinreste bzw. der Sequenzen SEQ ID No. 5, 11, 14, 18, 24 und 27 festgestellt werden. Erfindungsgemäß weist insbesondere die Glykierung des Lysinrests 141 in der SEQ ID No. 27 in Kombination mit dem HbA$_{1c}$-Spiegel eine hohe Sensitivität von 93,8% und eine hohe Selektivität von 97,9% für die Diagnose von Typ-2-Diabetes auf.

Daher eignen sich die Glykierungspositionen zur Verwendung als Biomarkerfür die Diagnostik von Diabetes, insbesondere Typ-II-Diabetes mellitus und/oder für die Überwachung einer Therapie von Diabetes, insbesondere Typ-II-Diabetes mellitus. Die Glykierung dieser 6 Lysine bzw. Sequenzen gemäß SEQ-ID No. 5, 11, 14, 18, 24 und 27 ist ein Zeichen für eine beginnende Diabeteserkrankung.

In einer weiteren Ausführungsform der Erfindung erfolgt im hyperglykämischen Zustand bei Typ-II-Diabetes-Patienten eine spezifische Glykierung der Lysinreste

-   Lys 93 (mit und ohne Met(O)) in Humanem Serum Albumin (P02768, SEQ ID No. 31),
-   Lys 181 in Humanem Serum Albumin (P02768, SEQ ID No. 31),
-   Lys 233 in Humanem Serum Albumin (P02768, SEQ ID No. 31),
-   Lys 378 in Humanem Serum Albumin (P02768, SEQ ID No. 31),
-   Lys 525 in Humanem Serum Albumin (P02768, SEQ ID No. 31),

- Lys 545 in Humanem Serum Albumin (P02768, SEQ ID No. 31),
- Lys 120 im Humanen Apolipoprotein A-I (P02647, SEQ ID No. 37),

bzw. in den Glykierungspositionen der Sequenzen gemäß SEQ ID No. 3, 6, 7, 10, 12, 23 und 25. Im Vergleich dazu konnte in einer Kontrollgruppe keinerlei Glykierung in den Glykierungspositionen der oben genannten Lysinreste bzw. der Sequenzen SEQ ID No. 3, 6, 7, 10, 12, 23 und 25 festgestellt werden.

Daher eignen sich die Glykierungspositionen zur Verwendung als Biomarkerfür die frühzeitige Bestimmung von Diabetes, insbesondere Typ-II-Diabetes mellitus vor der Manifestierung gesundheitlicher Beeinträchtigungen. Die Glykierung dieser 7 Lysine bzw. Sequenzen gemäß SEQ-ID No. 3, 6, 7, 10, 12, 23 und 25 ist ein Zeichen für eine beginnende Diabeteserkrankung.

In einer weiteren Ausführungsform umfasst das Verfahren die Schritte:

- Separation der Plasmaproteine einer Blutprobe,
- Durchführung eines enzymatischen Verdaus der Plasmaproteine,
- Bestimmung des Glykierungszustands zumindest eines der folgenden Lysinreste ausgewählt aus den SEQ ID No. 1 bis 30 und 44 bis 46, bevorzugt ausgewählt aus

    - Lys 174 in Humanem Serum Albumin (P02768, SEQ ID No. 31),
    - Lys 414 in Humanem Serum Albumin (P02768, SEQ ID No. 31),
    - Lys 574 in Humanem Serum Albumin (P02768, SEQ ID No. 31),
    - Lys 163 in der Humanen Fibrinogen beta-Kette (P02675, SEQ ID No. 33),
    - Lys 50 in der Humanen Ig Lambda Kette-C-Region (P01842; SEQ ID No. 36),
    - Lys 141 von Humanem Haptoglobin (P00738; SEQ ID No. 38)
    oder eines Peptids ausgewählt aus den SEQ ID No. 1 bis 30 und 44 bis 46, bevorzugt aus den SEQ-ID No. 5, 11, 14, 18, 24 und 27, besonders bevorzugt in der SEQ ID No. 27
    - sowie Bestimmung des $HbA_{1c}$-Spiegels, wobei nachfolgend die Glykierung in den Glykierungsposition mit dem $HbA_{1c}$-Spiegel korreliert wird.

In einer weiteren Ausführungsform umfasst das Verfahren die Schritte:

- Separation der Plasmaproteine einer Blutprobe,
- bevorzugt: Durchführung eines enzymatischen Verdaus der Plasmaproteine,
- Bestimmung des Glykierungszustands zumindest eines der folgenden Lysinreste ausgewählt aus den SEQ ID No. 1 bis 30 und 44 bis 46, bevorzugt ausgewählt aus
- Lys 93 (mit und ohne Met(O)) in Humanem Serum Albumin (P02768, SEQ ID No. 31),
- Lys 181 in Humanem Serum Albumin (P02768, SEQ ID No. 31),
- Lys 233 in Humanem Serum Albumin (P02768, SEQ ID No. 31),
- Lys 378 in Humanem Serum Albumin (P02768, SEQ ID No. 31),
- Lys 525 in Humanem Serum Albumin (P02768, SEQ ID No. 31),
- Lys 545 in Humanem Serum Albumin (P02768, SEQ ID No. 31),
- Lys 120 im Humanen Apolipoprotein A-I (P02647, SEQ ID No. 37),

    - oder eines Peptids ausgewählt aus den SEQ ID No. 1 bis 30 und 44 bis 46, bevorzugt aus den SEQ-ID No. 3, 6, 7, 10, 12, 23 und 25
    - sowie Bestimmung des $HbA_{1c}$-Spiegels, wobei nachfolgend die Glykierung in den Glykierungsposition mit dem $HbA_{1c}$-Spiegel korreliert wird

Die Separation der Plasmaproteine erfolgt dabei beispielsweise mittels Zentrifugation. Für den enzymatischen Verdau eignen sich grundsätzlich alle Proteasen, die einen Abbau der Plasmaproteine gewährleisten. Beispielsweise kann für den Verdau Trypsin verwendet werden.

In einer Variante der Erfindung wird nach dem enzymatischen Verdau eine Affinitätschromatographie zur Trennung glykierter Peptide und/oder eine Festphasenextraktion durchgeführt. Für die Affinitätschromatographie wird beispielsweise eine Säule oder bevorzugt magnetische Partikel verwendet. In einer Ausführungsform der Erfindung wird als Affinitätschromatographie eine Boronsäurechromatographie durchgeführt.

Dabei kommt es zu einer spezifischen Interaktion den cis-Diol-Gruppen der Zuckerreste in den Amadori-Peptiden und der Boronsäure. Daher eignet sich die Boronsäurechromatographie besonders für die effektive Anreicherung von Amadori-Peptiden.

Bevorzugt erfolgt die Bestimmung des Glykierungszustands mittels Massenspektrometrie, FRET (Förster-Energie-Transfer), ELBIA (Enzym-Linked-Boronate-Immunoassay) oder Immunoassay.

In einer weiteren Ausführungsform der Erfindung wird ein Verfahren zur nichtinvasiven Diagnostik von Diabetes, insbesondere Typ-II-Diabetes mellitus, angegeben, wobei die Glykierung von humanen Plasmaproteinen in zumindest einer Glykierungsposition ausgewählt aus

- Lys 557 in Humanem Serum Albumin (P02768, SEQ ID No. 31),
- Lys 75 in der Humanen Ig Kappa Kette-C-Region (P01834, SEQ ID No. 32),
- Lys 131 im Humanen Apolipoprotein A-I (P02647, SEQ ID No. 37),
- Lys 163 in der Humanen Fibrinogen beta-Kette (P02675, SEQ ID No. 33), und
- Lys 1162 von Humanem Alpha-2-macroglobulin (P01023, SEQ ID No. 34),
- sowie Bestimmung des HbA$_{1c}$-Spiegels erfolgt, wobei nachfolgend die Glykierung in den Glykierungsposition mit dem HbA$_{1c}$-Spiegel korreliert wird.

Die P-Nummer hinter dem Proteinnamen ist der UniProt Identifier. Die Sequenzpositionen beziehen sich auf die Sequenzen die jeweils im beiliegenden Sequenzprotokoll unter der in der Klammer genannten SEQ ID hinterlegt sind. Die Position im Humanen Serum Albumin bezieht sich dabei auf das reife Protein ohne das im UniProt-Eintrag angegebene Signalpeptid und ohne Propeptid.

Bevorzugt erfolgt die Bestimmung der Glykierung an zwei bis fünf, besonders bevorzugt drei bis fünf, weiter bevorzugt alle fünf der oben genannten Glykierungspositionen.

Bevorzugt wird die Glykierung in mindestens einer Sequenz ausgewählt aus den SEQ ID-No. 16, 18, 22, 26 oder 44 bestimmt. Bevorzugt erfolgt die Bestimmung der Glykierung in zwei bis fünf, besonders bevorzugt drei bis fünf, weiter bevorzugt alle fünf der SEQ ID-No. 16, 18, 22, 26 oder 44.

Die Sequenzen der erfindungsgemäßen Glykierungspositionen der humanen Plasmaproteine sind der nachfolgenden Tabelle 2 wiedergegeben:

Tab. 2

| SEQ-ID No. | Sequenz | Protein Annotation | Position[b] |
|---|---|---|---|
| 44 | AVMDDFAAFVEK*C*C*K | Humanes Serum Albumin (P02768) | K557 |
| 16 | DSTYSLSSTLTLSK*ADYEK | Ig kappa Kette C-Region (P01834) | K75 |
| 26 | K*WQEEMELYR | Apolipoprotein A-I (P02647) | K131 |
| 18 | QVK*DNENVVNEYSSELEK | Fibrinogen beta Kette (P02675) | K163 |
| 22 | ALLAYAFALAGNQDK*R | Alpha-2-macroglobulin (P01023) | K1162 |

Die Glykierung erfolgt dabei an die in der Tab. 1 angegebene Position der Lysinreste (K*) der Plasmaproteine. Bevorzugt wird durch Glykierung am Lysin (K) jeweils ein Amadori Produkt gebildet. Die Sequenz mit der SEQ ID No. 44 kann zusätzlich an einem oder mehreren Cysteinen (C*) in Folge des verwendeten Protokolls zur enzymatischen Spaltung der Plasma- oder Serumprobe alkyliert sein. Optional ist der Schwefel in den Methioninen Met3 in SEQ ID No. 44 und/oder Met6 in SEQ ID No. 26 jeweils (zum Sulfoxid) oxidiert.

Überaschenderweise wurde festgestellt, dass im hyperglykämischen Zustand bei Typ-II-Diabetes-Patienten eine spezifische Glykierung der Lysinreste

- Lys 557 in Humanem Serum Albumin (P02768, SEQ ID No. 31),
- Lys 75 in der Humanen Ig Kappa Kette-C-Region (P01834, SEQ ID No. 32),
- Lys 131 im Humanen Apolipoprotein A-I (P02647, SEQ ID No. 37),
- Lys 163 in der Humanen Fibrinogen beta-Kette (P02675, SEQ ID No. 33), und
- Lys 1162 von Humanem Alpha-2-macroglobulin (P01023, SEQ ID No. 34

bzw. in den Glykierungspositionen der Sequenzen gemäß SEQ ID No. 16, 18, 22, 26 oder 44 erfolgt. Im Vergleich dazu konnte in einer Kontrollgruppe keinerlei Glykierung in den Glykierungspositionen der oben genannten Lysinreste bzw. der Sequenzen SEQ ID No. 16, 18, 22, 26 oder 44 festgestellt werden. Daher eignen sich die Glykierungspositionen zur Verwendung als Biomarker für die Diagnostik von Diabetes, insbesondere Typ-II-Diabetes mellitus und/oder für die Überwachung einer Therapie von Diabetes, insbesondere Typ-II-Diabetes mellitus. Die Glykierung dieser 5 Lysine bzw. Sequenzen gemäß SEQ-ID No. 16, 18, 22, 26 oder 44 ist ein Zeichen für eine beginnende Diabeteserkrankung.

In einer weiteren Ausführungsform umfasst das Verfahren die Schritte:

- Separation der Plasmaproteine einer Blutprobe,
- bevorzugt: Durchführung eines enzymatischen Verdaus der Plasmaproteine,
- Bestimmung des Glykierungszustands zumindest eines der folgenden Lysinreste ausgewählt aus:

  - Lys 557 in Humanem Serum Albumin (P02768, SEQ ID No. 31),
  - Lys 75 in der Humanen Ig Kappa Kette-C-Region (P01834, SEQ ID No. 32),
  - Lys 131 im Humanen Apolipoprotein A-I (P02647, SEQ ID No. 37),
  - Lys 163 in der Humanen Fibrinogen beta-Kette (P02675, SEQ ID No. 33), und
  - Lys 1162 von Humanem Alpha-2-macroglobulin (P01023, SEQ ID No. 34
    oder eines Peptids ausgewählt aus einer Sequenz aus den SEQ ID No. 16, 18, 22, 26 oder 44

- sowie Bestimmung des $HbA_{1c}$-Spiegels, wobei nachfolgend die Glykierung in den Glykierungsposition mit dem $HbA_{1c}$-Spiegel korreliert wird.

Die Separation der Plasmaproteine erfolgt dabei beispielsweise mittels Zentrifugation. Für den enzymatischen Verdau eignen sich grundsätzlich alle Proteasen, die einen Abbau der Plasmaproteine gewährleisten. Beispielsweise kann für den Verdau Trypsin verwendet werden.

In einer Variante der Erfindung wird nach dem enzymatischen Verdau eine Affinitätschromatographie zur Trennung glykierter Peptide und/oder eine Festphasenextraktion durchgeführt. Für die Affinitätschromatographie wird beispielsweise eine Säule oder bevorzugt magnetische Partikel verwendet. In einer Ausführungsform der Erfindung wird als Affinitätschromatographie eine Boronsäurechromatographie durchgeführt. Dabei kommt es zu einer spezifischen Interaktion den cis-Diol-Gruppen der Zuckerreste in den Amadori-Peptiden und der Boronsäure. Daher eignet sich die Boronsäurechromatographie besonders für die effektive Anreicherung von Amadori-Peptiden.

Bevorzugt erfolgt die Bestimmung des Glykierungszustands mittels Massenspektrometrie, FRET (Förster-Energie-Transfer), ELBIA (Enzym-Linked-Boronate-Immunoassay) oder Immunoassay.

Bevorzugt umfasst das Verfahren zusätzlich die Bestimmung des Glykierungszustands zumindest ein der folgenden Lysinreste ausgewählt aus:

| Protein | Position | SEQ ID No. |
|---|---|---|
| Humanes Serum Albumin (P02768) | K359 | 31 |
| Humanes Serum Albumin (P02768) | K262 | 31 |

| | | |
|---|---|---|
| Humanes Serum Albumin (P02768) | K64 | 31 |
| Humanes Serum Albumin (P02768) | K181 | 31 |
| Humanes Serum Albumin (P02768) | K174 | 31 |
| Humanes Serum Albumin (P02768) | K51 | 31 |
| Humanes Serum Albumin (P02768) | K557 | 31 |
| Humanes Serum Albumin (P02768) | K378 | 31 |
| Humanes Serum Albumin (P02768) | K233 | 31 |
| Humanes Serum Albumin (P02768) | K545 | 31 |
| Humane Ig Kappa Kette-C-Region (P01834) | K99 | 32 |
| Humane Ig Kappa Kette-C-Region (P01834) | K41 | 32 |
| Humanes Apolipoprotein A-I (P02647) | K120 | 37 |
| Humanes Serotransferrin (P02787) | K683 | 35 |
| Humane Fibrinogen beta-Kette (P02675) | K211 | 33 |

Die Sequenzpositionen beziehen sich auf die Sequenzen, die jeweils im beiliegenden Sequenzprotokoll unter der in der Klammer genannten SEQ ID hinterlegt sind. Die Positionen im Humanen Serum Albumin beziehen sich dabei auf das reife Protein ohne das im UniProt-Eintrag angegebene Signalpeptid und ohne Propeptid.

Bevorzugt erfolgt die Bestimmung der Glykierung an zwei bis fünfzehn, besonders bevorzugt fünf bis zehn der oben genannten Glykierungspositionen.

Dazu erfolgt bevorzugt die Bestimmung des Glykierungszustands zumindest eines Peptids ausgewählt aus einer Sequenz aus den SEQ ID-No. 1 bis 30 und 44 bis 46 (s. auch Tabelle 1 und 2). Die Glykierung erfolgt dabei an die in die Tab. 1 oder 2 angegebenen Position der Lysinreste (K*) der Plasmaproteine. Bevorzugt wird durch Glykierung am Lysin (K) jeweils ein Amadori Produkt gebildet. Die Sequenzen können zusätzlich an einer oder mehreren der mit C* markierten Cysteine in Folge des verwendeten Protokolls zur enzymatischen Spaltung der Plasma- oder Serumprobe alkyliert sein. Optional ist der Schwefel in den Methioninen jeweils (zum Sulfoxid) oxidiert. Es wurde festgestellt, dass im hyperglykämischen Zustand bei Diabetes, insbesondere Typ-II-Diabetes mellitus Patienten eine signifikant erhöhte Glykierung in den Glykierungspositionen in den Sequenzen SEQ-ID No. 1 bis 30 und 44 bis 46 vorliegt. Daher eignen sich die Sequenzen SEQ-ID No. 1 bis 30 und 44 bis 46, bevorzugt zumindest die SEQ ID No. 27 zur Stützung der Diagnose basierend auf der Bestimmung der Glykierung in zumindest einer Glykierungsposition ausgewählt aus den Sequenzen SEQ-ID No. 1 bis 30 und 44 bis 46. Die Sequenzen der SEQ-ID No. 1 bis 30 und 44 bis 46 sind der Tab. 1 und 2 zu entnehmen. Das Verfahren beinhaltet dabei die Bestimmung der Glykierung in zumindest einer Position ausgewählt aus den Sequenzen SEQ-ID No. 1 bis 30 und 44 bis 46 und Vergleich der Glykierung mit einem Kontrollwert sowie mit dem Wert des HbA$_{1c}$-Spiegels, wobei nachfolgend die Glykierung in den Glykierungspositionen mit dem HbA$_{1c}$-Spiegel korreliert wird. Bei Überschreiten des Kontrollwerts ist das Vorliegen von Diabetes, insbesondere Typ-II-Diabetes mellitus, festgestellt.

Bevorzugt wird die Glykierung in mindestens einer Sequenz ausgewählt aus den SEQ ID-No. 1 bis 30 sowie 44 bis 46, bevorzugt zumindest in der SEQ ID No. 27 bestimmt. Bevorzugt erfolgt die Bestimmung der Glykierung in zwei bis fünfzehn, besonders bevorzugt fünf bis zehn der SEQ ID-No. 1 bis 30 sowie 44 bis 46.

Gegenstand der Erfindung sind auch die Sequenzen SEQ ID-No. 1 bis 30 und 44 bis 46 sowie die Verwendung eines glykierten Lysins ausgewählt aus:

- Lys 174 in Humanem Serum Albumin (P02768, SEQ ID No. 31),
- Lys 414 in Humanem Serum Albumin (P02768, SEQ ID No. 31),
- Lys 574 in Humanem Serum Albumin (P02768, SEQ ID No. 31),
- Lys 163 in der Humanen Fibrinogen beta-Kette (P02675, SEQ ID No. 33),
- Lys 50 in der Humanen Ig Lambda Kette-C-Region (P01842; SEQ ID No. 36),
- Lys 141 von Humanem Haptoglobin (P00738; SEQ ID No. 38)
- Lys 93 (mit und ohne Met(O)) in Humanem Serum Albumin (P02768, SEQ ID No. 31),
- Lys 181 in Humanem Serum Albumin (P02768, SEQ ID No. 31),
- Lys 233 in Humanem Serum Albumin (P02768, SEQ ID No. 31),
- Lys 378 in Humanem Serum Albumin (P02768, SEQ ID No. 31),
- Lys 525 in Humanem Serum Albumin (P02768, SEQ ID No. 31),
- Lys 545 in Humanem Serum Albumin (P02768, SEQ ID No. 31),
- Lys 120 im Humanen Apolipoprotein A-I (P02647, SEQ ID No. 37)

bzw. in den Glykierungspositionen der Sequenzen gemäß SEQ ID No. 5, 11, 14, 18, 24, 27, 3, 6, 7, 10, 12, 23 und 25 zur nichtinvasiven Diagnostik von Diabetes, insbesondere Typ-II-Diabetes mellitus.

Gegenstand der Erfindung sind auch die Sequenzen SEQ ID-No. 1 bis 30 und 44 bis 46 sowie die Verwendung eines glykierten Lysins ausgewählt aus:

- Lys 174 in Humanem Serum Albumin (P02768, SEQ ID No. 31),
- Lys 414 in Humanem Serum Albumin (P02768, SEQ ID No. 31),
- Lys 574 in Humanem Serum Albumin (P02768, SEQ ID No. 31),
- Lys 163 in der Humanen Fibrinogen beta-Kette (P02675, SEQ ID No. 33),
- Lys 50 in der Humanen Ig Lambda Kette-C-Region (P01842; SEQ ID No. 36),
- Lys 141 von Humanem Haptoglobin (P00738; SEQ ID No. 38)
- Lys 93 (mit und ohne Met(O)) in Humanem Serum Albumin (P02768, SEQ ID No. 31),
- Lys 181 in Humanem Serum Albumin (P02768, SEQ ID No. 31),
- Lys 233 in Humanem Serum Albumin (P02768, SEQ ID No. 31),
- Lys 378 in Humanem Serum Albumin (P02768, SEQ ID No. 31),
- Lys 525 in Humanem Serum Albumin (P02768, SEQ ID No. 31),
- Lys 545 in Humanem Serum Albumin (P02768, SEQ ID No. 31),
- Lys 120 im Humanen Apolipoprotein A-I (P02647, SEQ ID No. 37),

bzw. in den Glykierungspositionen der Sequenzen gemäß SEQ ID No. 5, 11, 14, 18, 24, 27, 3, 6, 7, 10, 12, 23 und 25 für die Überwachung einer Therapie von Diabetes, insbesondere Typ-II-Diabetes mellitus,

- sowie Bestimmung des HbA$_{1c}$-Spiegels, wobei nachfolgend die Glykierung in den Glykierungsposition mit dem HbA$_{1c}$-Spiegel korreliert wird.

Ebenfalls Gegenstand der Erfindung ist ein Kit zur nichtinvasiven Diagnostik von Diabetes, insbesondere Typ-II-Diabetes mellitus, umfassend zumindest ein Reagenz, welches eine Affinität zu zumindest einem Antigen aufweist, welches durch ein Peptid gebildet wird, welches die Glykierungsposition Lys 141 von Humanem Haptoglobin (P00738; SEQ ID No. 38) aufweist.

Bevorzugt hat das Antigen eine Länge von 7 bis 25 Aminosäureresten, Besonders bevorzugt wird das Antigen durch eine Sequenz ausgewählt aus den Sequenzen SEQ ID No. 1 bis 30 und 44 bis 46 gebildet, besonders bevorzugt ausgewählt aus den SEQ ID No. 5, 11, 14, 18, 24, 27, 3, 6, 7, 10, 12, 23 und 25.

Erfindungsgemäß wird das Antigen im glykierten Zustand Zustand detektiert.

Das Reagenz hat spezifische Bindungseigenschaften für das Antigen. In einer Ausführungsform der Erfindung ist das Reagenz ein Antikörper, Oligonukleotid-Aptamer oder Peptid-Aptamer. Der Begriff Antikörper umfasst rekombinant hergestellte Antikörper Fragmente, wie z.B. scFV-Fragmente.

In einer Ausführungsform der Erfindung umfasst das Kit weiterhin zumindest eine immobilisierte Boronsäurekomponente zur Anreicherung von glykierten Proteinen und Peptiden. Dabei kann in einem ersten Schritt eine Trennung von glykierten Proteinen und Peptiden (Amadori-Proteinen oder Amadori-Peptiden) durch spezifische Interaktion der Boronsäure mit den cis-diol-Gruppen der Zuckerreste in den Amadori-Proteinen oder Amadori-Peptiden erfolgen. Anschließend erfolgt eine Bestimmung der Amadori-Proteine oder Amadori-Peptide (insbesondere der SEQ ID No. 5, 11, 14, 18, 24, 27, 3, 6, 7, 10, 12, 23 und 25) mittels Antikörper.

Eine Anreicherung ist bei der Verwendung von besonders sensitiven Techniken, wie z. B. Tandemmassenspektrometrie nicht zwingend notwendig.

In einer weiteren Ausführungsform der Erfindung umfasst das Kit einen ELBIA (Enzym-Linked-Boronate-Immunoassay). Dabei wird beispielsweise zunächst zumindest ein Antikörper gegen eine Sequenz ausgewählt aus den SEQ-ID No.1 bis 30 und 44 bis 46 in einem Reaktionsgefäß (96-well-Platte; Mikrogefäß (1,5 mL)) immobilisiert. Anschließend wird eine zu testende Probe in das Reaktionsgefäß eingebracht, wobei es zu einer spezifischen Interaktion zwischen Antikörper und Antigen kommt, wobei das Antigen wie oben beschreiben ausgewählt ist. Die nicht gebundenen Peptide werden durch Waschen entfernt. Anschließend wird ein Boronsäure-Konjugat, beispielsweise Boronsäure-Peroxidase-Konjugat, zugegeben und eine Peroxidasereaktion durch weitere Zugabe von o-Phenylendiamin und H$_2$O$_2$ initiiert. Der Verlauf der Reaktion kann dabei photometrisch bei 492 nm verfolgt werden.

Gegenstand der Erfindung ist ebenfalls die Verwendung eines glykierten Lysins ausgewählt aus den SEQ ID No. 1 bis 30 und 44 bis 46, bevorzugt ausgewählt aus:

- Lys 174 in Humanem Serum Albumin (P02768, SEQ ID No. 31),
- Lys 414 in Humanem Serum Albumin (P02768, SEQ ID No. 31),
- Lys 574 in Humanem Serum Albumin (P02768, SEQ ID No. 31),
- Lys 163 in der Humanen Fibrinogen beta-Kette (P02675, SEQ ID No. 373),
- Lys 50 in der Humanen Ig Lambda Kette-C-Region (P01842; SEQ ID No. 36),
- Lys 141 von Humanem Haptoglobin (P00738; SEQ ID No. 38)
- Lys 93 (mit und ohne Met(O)) in Humanem Serum Albumin (P02768, SEQ ID No. 31),
- Lys 181 in Humanem Serum Albumin (P02768, SEQ ID No. 31),
- Lys 233 in Humanem Serum Albumin (P02768, SEQ ID No. 31),
- Lys 378 in Humanem Serum Albumin (P02768, SEQ ID No. 31),
- Lys 525 in Humanem Serum Albumin (P02768, SEQ ID No. 31),
- Lys 545 in Humanem Serum Albumin (P02768, SEQ ID No. 31),
- Lys 120 im Humanen Apolipoprotein A-I (P02647, SEQ ID No. 37),

bzw. in den Glykierungspositionen der Sequenzen gemäß SEQ ID No. 5, 11, 14, 18, 24, 27, 3, 6, 7, 10, 12, 23 und 25, bevorzugt SEQ ID No. 27 als Biomarker für die Diagnostik von Diabetes, insbesondere Typ-II-Diabetes mellitus und/oderfür die Überwachung einer Therapie von Diabetes, insbesondere Typ-II-Diabetes mellitus.

Gegenstand der Erfindung ist ebenfalls ein Biomarker umfassend zumindest ein glykiertes Lysin ausgewählt aus den SEQ ID No. 1 bis 30 und 44 bis 46, bevorzugt ausgewählt aus:

- Lys 174 in Humanem Serum Albumin (P02768, SEQ ID No. 31),
- Lys 414 in Humanem Serum Albumin (P02768, SEQ ID No. 31),
- Lys 574 in Humanem Serum Albumin (P02768, SEQ ID No. 31),

- Lys 163 in der Humanen Fibrinogen beta-Kette (P02675, SEQ ID No. 373),
- Lys 50 in der Humanen Ig Lambda Kette-C-Region (P01842; SEQ ID No. 36),
- Lys 141 von Humanem Haptoglobin (P00738; SEQ ID No. 38)
- Lys 93 (mit und ohne Met(O)) in Humanem Serum Albumin (P02768, SEQ ID No. 31),
- Lys 181 in Humanem Serum Albumin (P02768, SEQ ID No. 31),
- Lys 233 in Humanem Serum Albumin (P02768, SEQ ID No. 31),
- Lys 378 in Humanem Serum Albumin (P02768, SEQ ID No. 31),
- Lys 525 in Humanem Serum Albumin (P02768, SEQ ID No. 31),
- Lys 545 in Humanem Serum Albumin (P02768, SEQ ID No. 31),
- Lys 120 im Humanen Apolipoprotein A-I (P02647, SEQ ID No. 37),

bzw. in den Glykierungspositionen der Sequenzen gemäß SEQ ID No. 5, 11, 14, 18, 24, 27, 3, 6, 7, 10, 12, 23 und 25, bevorzugt SEQ ID No. 27 für die Diagnostik von Diabetes, insbesondere Typ-II-Diabetes mellitus und/oder für die Überwachung einer Therapie von Diabetes, insbesondere Typ-II-Diabetes mellitus.

Gegenstand der Erfindung ist ebenfalls ein Verfahren zur Diagnose von Diabetes insbesondere Typ-II-Diabetes mellitus umfassend die Schritte:

- Separation der Plasmaproteine einer Blutprobe,

- Durchführung eines enzymatischen Verdaus der Plasmaproteine,

- Bestimmung des Glykierungszustands zumindest eines der folgenden Lysinreste ausgewählt aus den SEQ ID No. 1 bis 30 und 44 bis 46, bevorzugt ausgewählt aus

  - Lys 174 in Humanem Serum Albumin (P02768, SEQ ID No. 31),
  - Lys 414 in Humanem Serum Albumin (P02768, SEQ ID No. 31),
  - Lys 574 in Humanem Serum Albumin (P02768, SEQ ID No. 31),
  - Lys 163 in der Humanen Fibrinogen beta-Kette (P02675, SEQ ID No. 373),
  - Lys 50 in der Humanen Ig Lambda Kette-C-Region (P01842; SEQ ID No. 36),
  - Lys 141 von Humanem Haptoglobin (P00738; SEQ ID No. 38)
  - Lys 93 (mit und ohne Met(O)) in Humanem Serum Albumin (P02768, SEQ ID No. 31),
  - Lys 181 in Humanem Serum Albumin (P02768, SEQ ID No. 31),
  - Lys 233 in Humanem Serum Albumin (P02768, SEQ ID No. 31),
  - Lys 378 in Humanem Serum Albumin (P02768, SEQ ID No. 31),
  - Lys 525 in Humanem Serum Albumin (P02768, SEQ ID No. 31),
  - Lys 545 in Humanem Serum Albumin (P02768, SEQ ID No. 31),
  - Lys 120 im Humanen Apolipoprotein A-I (P02647, SEQ ID No. 37),

bzw. in den Glykierungspositionen der Sequenzen gemäß SEQ ID No. 5, 11, 14, 18, 24, 27, 3, 6, 7, 10, 12, 23 und 25, bevorzugt SEQ ID No. 27

Überraschend wurde festgestellt, dass eine Korrelation der Bestimmung des Glykierungswertes in einer Glykierungsposition ausgewählt aus den SEQ ID No. 1 bis 30 und 44 bis 46 mit dem $HbA_{1c}$-Spiegel eine hohe Selektivität erzielt werden kann. Dabei kann mittels Hauptkomponentenanalyse eine Struktur bzw. ein Muster in einem Datensatz von Probanden erkannt werden, wobei sich nach voriger Kategorisierung der einzelnen Patienten zeigte, dass ein gewisser Prozentsatz der diabetischen Patienten aufgrund bestimmter Parameter ein anderes Verhalten aufweist, als die restlichen Probanden.

Die Hauptkomponentenanalyse oder englisch Principal Component Analysis (PCA) ist ein Verfahren der multivariaten Statistik. Sie dient dazu, umfangreiche Datensätze zu strukturieren, zu vereinfachen und zu veranschaulichen, indem eine Vielzahl statistischer Variablen durch eine geringere Zahl möglichst aussagekräftiger Linearkombinationen (die "Hauptkomponenten") genähert wird. Mathematisch wird eine Hauptachsentransformation durchgeführt: Man minimiert die Korrelation mehrdimensionaler Merkmale durch Überführung in einen Vektorraum mit neuer Basis. Die Hauptachsentransformation lässt sich durch eine orthogonale Matrix angeben, die aus den Eigenvektoren der Kovarianzmatrix gebildet wird. Die Hauptkomponentenanalyse ist damit problemabhängig, weil für jeden Datensatz eine eigene Transformationsmatrix berechnet werden muss. Die Rotation des Koordinatensystems wird dabei so ausgeführt, dass die Kovarianzmatrix diagonalisiert wird, d. h. die Daten werden dekorreliert (die Korrelationen sind die Nicht-diagonal-Einträge der Kovarianzmatrix). Für normalverteilte Datensätze bedeutet dies, dass die einzelnen Komponenten jedes Datensatzes nach der PCA voneinander statistisch unabhängig sind, da die Normalverteilung durch das nullte (Normierung), erste (Mittelwert) und zweite Moment (Kovarianzen) vollständig charakterisiert wird.

Die Hauptkomponentenanalyse (PCA) wird auch häufig in der Clusteranalyse und zur Reduzierung der Dimension des Parameterraums verwendet, insbesondere dann, wenn man noch keinerlei Vorstellung (Modell) von der Struktur der Daten hat. Dabei macht man sich zunutze, dass die PCA das (orthogonale) Koordinatensystem so dreht, dass die Kovarianzmatrix diagonalisiert wird. Außerdem sortiert die PCA die Reihenfolge der Koordinatenachsen (die Hauptkomponenten) so um, dass die erste Hauptkomponente den größten Anteil der Gesamtstreuung (Totale Varianz) im Datensatz enthält, die zweite Hauptkomponente den zweitgrößten Anteil, usw. Wie an den Beispielen im vorigen Abschnitt illustriert wurde, kann man meist die hinteren Hauptkomponenten (also diejenigen, welche nur einen geringen Anteil an der Gesamtstreuung enthalten) ersatzlos streichen, ohne dass dadurch ein nennenswerter Informationsverlust entsteht.

Die Grundannahme für die Verwendung der PCA zur Clusteranalyse und Dimensionsreduktion lautet: Die Richtungen mit der größten Streuung (Varianz) beinhalten die meiste Information.

Nachfolgend wurde eine Clusteranalyse vorgenommen, bei der angenommen wurde, dass sich Diabetiker bezüglich des Glykierungsmusters in drei Untergruppen differenzieren lassen. Dabei konnte gezeigt werden, dass die Reduktion des Datensatzes auf drei Dimensionen (Hauptkomponenten) eine Aufteilung der Diabetiker in drei Cluster zuließ, was sich mit den klinischen Beobachtungen deckt.

Unter einer Clusteranalyse (Clustering-Algorithmus, gelegentlich auch: Ballungsanalyse) versteht man Verfahren zur Entdeckung von Ähnlichkeitsstrukturen in (großen) Datenbeständen. Die so gefundenen Gruppen von "ähnlichen" Objekten werden als Cluster bezeichnet, die Gruppenzuordnung als Clustering. Die gefundenen Ähnlichkeitsgruppen können graphentheoretisch, hierarchisch, partitionierend oder optimierend sein.

Die Eigenschaften der zu untersuchenden Objekte werden mathematisch als Zufallsvariablen aufgefasst. Sie werden in der Regel in Form von Vektoren als Punkte in einem Vektorraum dargestellt, deren Dimensionen die Eigenschaftsausprägungen des Objekts bilden. Bereiche, in denen sich Punkte anhäufen (Punktwolke), werden Cluster genannt. Bei Streudiagrammen dienen die Abstände der Punkte zueinander oder die Varianz innerhalb eines Clusters als sogenannte Proximitätsmaße, welche die Ähnlichkeit bzw. Unterschiedlichkeit zwischen den Objekten zum Ausdruck bringen.

Ein Cluster kann auch als eine Gruppe von Objekten definiert werden, die in Bezug auf einen berechneten Schwerpunkt eine minimale Abstandssumme haben. Dazu ist die Wahl eines Distanzmaßes erforderlich. In bestimmten Fällen sind die Abstände (bzw. umgekehrt die Ähnlichkeiten) der Objekte untereinander direkt bekannt, so dass sie nicht aus der Darstellung im Vektorraum ermittelt werden müssen.

Zur Durchführung der Clusteranalyse an 50 Typ-2-Diabetes Patienten wurde vorliegend der Expectation-Maximization-Algorithmus (kurz EM-Algorithmus) verwendet. Dabei wurden fehlende Werte durch Mittelwerte des korrespondierenden Merkmalsvektor ersetzt. Der Merkmalsraum beinhaltet dabei etwa Alter, Gewicht, Größe, Proteinabundanz, Profil, etc. Die Methode beinhaltet dabei die Annahme, dass die Daten einer Multivarianten Normalverteilung unterliegen. Gestützt auf bisherige Kenntnisse wurde eine Clusteranzahl von drei angenommen. Die Ergebnisse zeigen dabei eine sehr gute Verteilung der Cluster. Zudem wurde ein Clusterstabilitätstest nach dem "elbow criterion" vorgenommen, bei dem gezeigt werden konnte, dass die maximale Anzahl an erhaltenen Clustern drei beträgt.

Die Kernidee des EM-Algorithmus ist es, mit einem zufällig gewählten Modell zu starten, und abwechselnd die Zuordnung der Daten zu den einzelnen Teilen des Modells (Expectation-Schritt) und die Parameter des Modells an die neueste Zuordnung (Maximization-Schritt) zu verbessern. In beiden Schritten wird dabei die Qualität des Ergebnisses verbessert: im E-Schritt werden die Punkte besser zugeordnet, im M-Schritt wird das Modell so verändert, dass es besser zu den Daten passt. Findet keine wesentliche Verbesserung mehr statt, beendet man das Verfahren.

Das EM-Clustering ist ein Verfahren zur Clusteranalyse, das die Daten mit einem "Mixture of Gaussians"-Modell - also als Überlagerung von Normalverteilungen - repräsentiert. Dieses Modell wird zufällig oder heuristisch initialisiert und anschließend mit dem allgemeinen EM-Prinzip verfeinert.

Zur Durchführung der Klassifizierung von 48 Typ-2-Diabetes Patienten und 48 Kontrollprobanden wurde der Entscheidungsbaum-Algorithmus verwendet. Der Merkmalsraum wurde dabei durch das Abundanzprofil von 27 glykierten Peptidsequenzen ausgewählt aus den SEQ ID No. 1 bis 30 und 44 bis 46 und dem $HbA_{1c}$ Wert gebildet. Der Entscheidungsbaum ist ein schrittweiser Greedy-Algorithmus. Greedy-Algorithmen bilden eine spezielle Klasse von Algorithmen, welche sich dadurch auszeichnen, dass sie schrittweise den Folgezustand auswählen, der zum Zeitpunkt der Wahl den größten Gewinn bzw. das beste Ergebnis verspricht (z. B. Gradientenverfahren). Um unter den Folgezuständen eine Auswahl zu treffen, wird oft eine Bewertungsfunktion verwendet. Vorliegend wurde in jedem Schritt ein Merkmal hinzugefügt, sofern eine Erhöhung der Genauigkeit erzielt wurde. So konnte beispielsweise Spezifität von 97,9% für eine Kombination von $HbA_{1c}$ und Lys-141 in Haptoglobin (SEQ ID No. 27) errechnet werden. Dabei konnte ausgehend von HbA1c (94% Spezifität & 77% Sensitivität) und Lys-141 in Haptoglobin (SEQ ID No. 27) (HP K141, 83% Spezifität & 60% Sensitivität) durch die Kombination beider Marker eine deutlich bessere Sensitivität von 93,8% und sogar eine noch höhere Spezifität von 97,9% für die Diagnose von Typ-2-Diabetes erreicht werden.

Zur Realisierung der Erfindung ist es auch zweckmäßig die vorbeschriebenen Ausführungsformen zu kombinieren.

Im Folgenden wird die Erfindung anhand einiger Ausführungsbeispiele und Figuren näher erläutert, ohne darauf beschränkt zu sein.

Fig. 1 zeigt eine schematische Darstellung der Quantifizierung ausgewählter glykierter Peptide,

Fig. 2 zeigt eine Korrelationsanalyse für die Korrelation von Gehalten an Amadori-Peptiden und den Werten für BMI und C-Peptid,

Fig. 3 eine schematische Darstellung einer Hauptkomponentenanalyse mit Unterteilung in drei Untergruppen,

Fig. 4 eine schematische Darstellung HbA$_{1C}$-Werte aufgetragen gegen den Glykierungsgrad von HbA$_{1c}$ und Lys-141 in Haptoglobin (SEQ ID No. 27),

Fig. 5 eine schematische Darstellung der ROC-Kurven für den Glykierungsgrad von Lys-141 in Haptoglobin (HP K141) und HbA1c und in

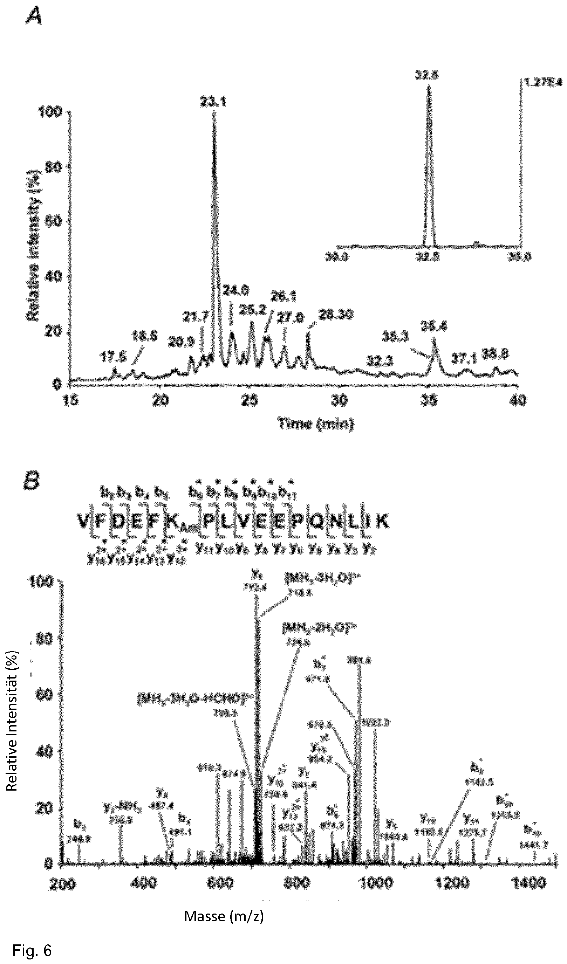

Fig. 6 eine schematische Darstellung der exemplarischen Bestimmung der glykierten Peptide mittels Tandemmassenspektrometrie.

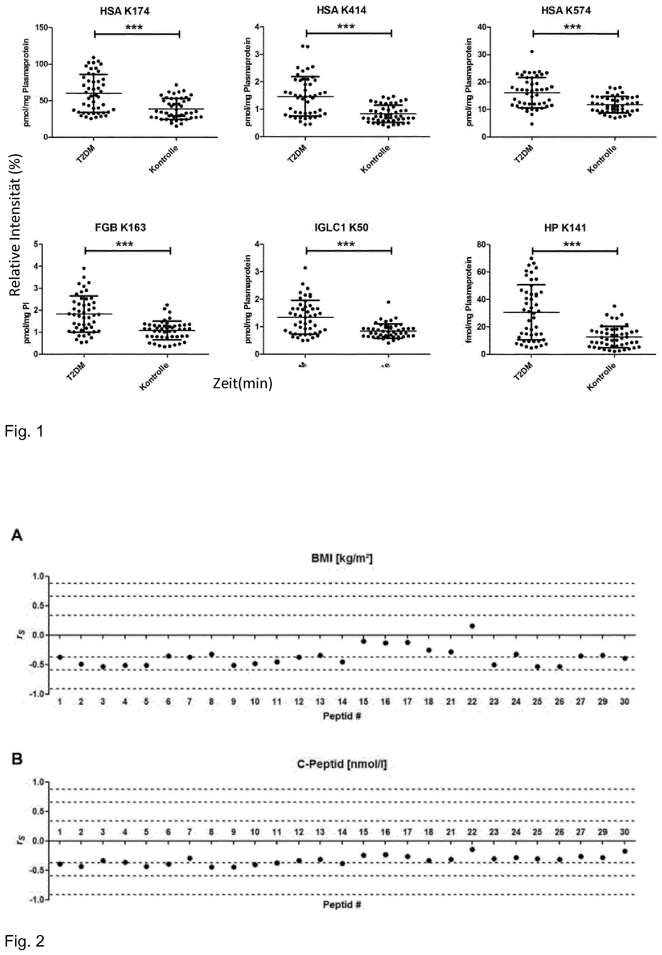

In einem ersten Ausführungsbeispiel ist in der Fig. 1 die Quantifizierung ausgewählter glykierter Peptide im tryptischen Verdau von Plasmaproben von Typ-2-Diabetikern und Kontrollprobanden dargestellt. Die Unterschiede zwischen den Kontrollprobanden und den Typ-2-Diabetikern waren dabei signifikant (p<0,0001).

In einem weiteren Ausführungsbeispiel ist in Fig. 2 eine Korrelationsanalyse nach Spearman dargestellt. Dabei ergibt sich Spearman's Korrelationskoeffizient (rs) (nummerische Werte als Punkte angedeutet) für die Korrelation von Gehalten an Amadori-Peptiden und den Werten für BMI (A) und C-Peptid (B) der einzelnen Probanden.

Der Rangkorrelationskoeffizient nach Spearman ist ein Maß für die Stärke eines monotonen Zusammenhangs zwischen zwei mindestens ordinal skalierten Größen. Im Gegensatz zum Korrelationskoeffizienten nach Pearson wird bei der Berechnung des Korrelationskoeffizienten nach Spearman kein linearer Zusammenhang vorausgesetzt. Voraussetzungen sind, dass die zu korrelierenden Größen mindestens ordinal skaliert sind, unabhängige Beobachtungspaare vorliegen und der untersuchte Zusammenhang monoton ist.

Für die Berechnung werden die Werte beider Merkmale zunächst für sich aufsteigend der Größe nach sortiert und mit entsprechenden Rangzahlen versehen. Anschließend wird für jedes Wertepaar (xi,yi) die Differenz di berechnet. Der Spearmansche Korrelationskoeffizient rs ergibt sich dann wie folgt:

$$r_s = 1 - \frac{6 \cdot \sum_{i=1}^{n} d_i^2}{n \cdot (n^2 - 1)}$$

Dabei ist n die Anzahl der Beobachtungspaare. Ist der Korrelationskoeffizient rs > 0, so liegt ein positiver Zusammenhang vor, ist rs < 0 so besteht ein negativer Zusammenhang. Kein Zusammenhang liegt vor, wenn rs = 0 ist. Der Korrelationskoeffizient rs nimmt Werte zwischen -1 und +1 an. Je dichter rs bei 0 liegt, desto schwächer ist der Zusammenhang, je näher $r_S$ bei -1 oder +1 liegt, desto stärker ist der Zusammenhang.

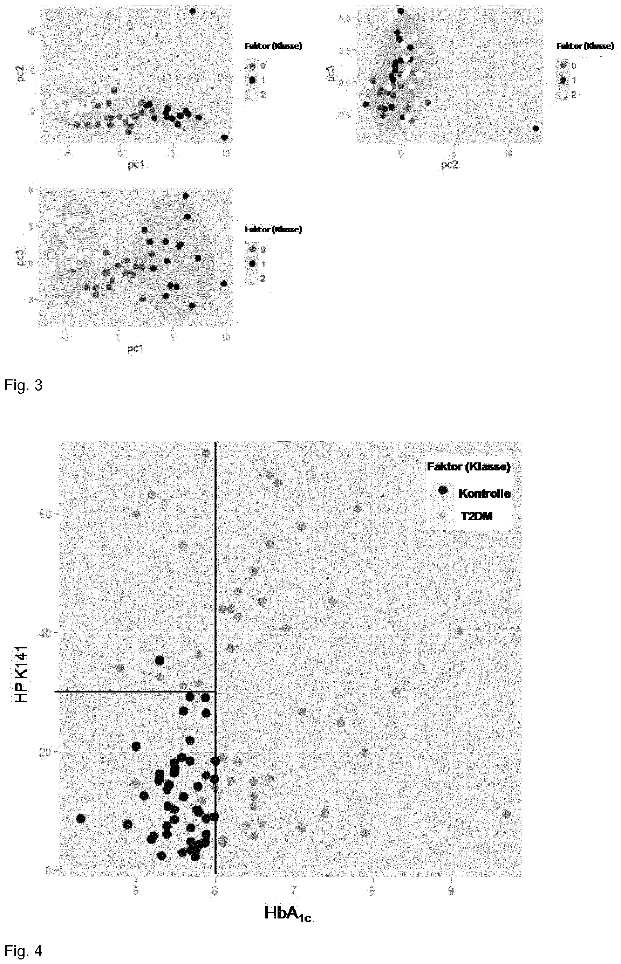

In einem weiteren Ausführungsbeispiel ist in Fig. 3 eine Clusteranalyse unter der Annahme, dass es drei Diabetes-Arten gibt dargestellt. Die sich ergebenden Cluster sind dabei als 0, 1 und 2 dargestellt und weisen jeweils für sich bestehende Ähnlichkeiten auf.

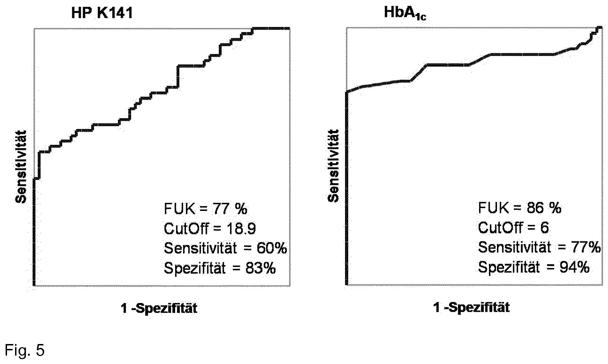

In einem weiteren Ausführungsbeispiel sind in Fig. 4 HbA$_{1c}$-Werte von Diabetikern und Kontrollprobanden aufgetragen gegen den Glykierungsgrad von HbA$_{1c}$ und Lys-141 in Haptoglobin. Dabei wurde überprüft, ob die Kombination verschiedener diagnostischer Parameter mit dem Glykierungsgrads der einzelnen Glykierungsstellen eine Erhöhung von Sensitivität und Selektivität für die Diagnose von Diabetes zufolge hat. Beispielsweise konnte durch die Kombination von HbA$_{1c}$ und Lys-141 in Haptoglobin (SEQ ID No. 27) (HP K141) eine deutlich bessere Sensitivität von 93,8% und sogar eine noch höhere Spezifität von 97,9% für die Diagnose von Typ-2-Diabetes erreicht werden. Die Fig. 5 zeigt dabei ROC-Kurven für den Glykierungsgrad von Lys-141 in Haptoglobin (SEQ ID No. 27) (HP K141) 83% Spezifität und 60% Sensitivität sowie für HbA$_{1c}$. 94% Spezifität und 77% Sensitivität. Die Receiver Operating Characteristic (ROC) - Kurve bzw. Grenzwertoptimierungskurve ist eine Methode zur Bewertung und Optimierung von Analyse-Strategien. Die ROC-Kurve stellt visuell die Abhängigkeit der Effizienz mit der Fehlerrate für verschiedene Parameterwerte dar. Sie ist eine Anwendung der Signalentdeckungstheorie.

In einem weiteren Ausführungsbeispiel wird die Bestimmung der Glykierung in den Glykierungspositionen der Sequenzen SEQ ID No.1 bis 30 beschrieben. Dabei werden Blutproben von Typ-II-Diabetes mellitus Patienten und Nichtdiabetespatienten zunächst zentrifugiert (9168 g, 30 min, 4°C, Allegra centrifuge 21R, Beckman Coulter, Krefeld). Der Überstand wird danach 10fach mit Ammoniumhydrogencarbonat (0,1 mol/L, pH 8,0) verdünnt und nachfolgend mittels Vivaspin Filter (Sartorius Stedim Biotech) entsalzt. Die erhaltene Proteinkonzentration wurde mittels Bradford-Assay bestimmt. Aliquots mit einem Proteingehalt von 25 μg mit SDS (10% in Wasser, w:v, 2 μL) und TCEP (Tris(2-carboxyethyl) phosphinhydrochlorid, 50 mmol/L) versetzt und mit wässriger Ammoniumhydrogencarbonatlösung (50 mmol/L) auf ein Endvolumen von 20 μL verdünnt und für 15 min bei 60°C inkubiert. Die Proben wurden auf Raumtemperatur abgekühlt und mit Iodacetamid (0,1 mol/L, 2,2 μL) unter Lichtausschluss alkyliert (15 min bei Raumtemperatur). Daran anschließend wurde ein enzymatischer Verdau mit Trypsin (25 mg/L in 50 mmol/L Ammoniumhydrogencarbonat, 50 μL) bei 37 °C über Nacht durchgeführt. Der vollständige Verdau wurde durch Prüfung der Bande des Humanen Serum Albumins mittels SDS-PAGE nachgewiesen.

Aliquots der verdauten Proben (20 μg) wurden mit kaltem Puffer (4°C, 50 mmol/L Magnesiumacetat; 250 mmol/L Ammoniumacetat, pH 8,1) auf ein Volumen von 300 μL verdünnt. Die Proben wurden auf eine Polypropylensäule (1 mL; Qiagen) gefüllt mit mAPBA (m-Aminophenylboronsäureagarose 1mL, Säulenbettvolumen) aufgebracht.

Die Amadori-Peptide wurden in zwei Schritten mit Essigsäure (0,1 mol/L, 7 mL und 0,2 mol/L, 1 mL) bei 37 °C eluiert. Die Eluate wurden lyophilisiert.

Die Lyophilisate wurden in einer Mischung aus wässriger Acetonitrillösung (20%, v/v, 12,5 μL) und Ameisensäure (0,1%, v/v, 87,5 μL) aufgenommen und zur Festphasenextraktion in eine C18-Gel-Pipettenspitze (Thermo Fisher Scientific) eingebracht, welche mit einer Lösung aus wässrigem Acetonitril (2,5%) enthaltend Ameisensäure (0,1%) (Eluent A) äquilibriert war. Die Gel-Pipettenspitze wurde mit 150 μL Eluent A gewaschen, bevor die Peptide mit einer wässrigen Acetonitrillösung (60%) enthaltend Ameisensäure (0,1%) (Eluent B) eluiert wurden.

Die Bestimmung der Glykierung in den Glykierungspositionen der Sequenzen SEQ-ID No. 1 bis 30 wurde mittels Massenspektrometrie vorgenommen. Dazu wurden die Proben in wässriger Acetonitrillösung (3%, v/v; 50 μL) gelöst. Aliquots (10 μL) wurden auf eine nanoAcquity UPLC Symmetry Trap-Säule (Waters GmbH) (5 μL/min, 5 min) aufgebracht und auf einer nano Acquity UPLC BEH130-Säule (Waters GmbH) (30°C) unter Verwendung eines nano Acquity UPLC Systems (Waters GmbH) getrennt. Die Analyten wurden mit einem linearen Gradienten von 3% auf 50 % (45 min) und von 50% auf 80% Eluent B (2 min) bei einer Flussrate von 0,4 μL/min eluiert. Die Analyse der Eluenten erfolgt mittels Tandemmassenspektrometrie (LTQ Orbitrap XL ETD; Thermo Fisher Scientific). Für die Zuordnung der Peptide wurden den Proben $^{13}$C-angereicherte Peptide zugesetzt und die Quantifizierung der nativ vorhandenen glykierten Peptide erfolgt gegenüber den zugesetzten Peptiden. Alternativ erfolgte die Zuordnung der Peptide mittels Abgleich mit der SwissProt Datenbank, wobei Amadori-Peptide manuell zugeordnet wurden. Ein Beispiel für eine solche Zuordnung ist in der Fig.1 wiedergegeben. Dabei zeigt die Fig. 5A ein Chromatogramm der UPLC mit den Retentionszeiten der einzelnen Analyten, währen die Fig. 5B eine Darstellung der mittels Tandemmassenspektometrie ermittelten Massenfragmente der einzelnen Verbindungen und deren Zuordnung zu einer Sequenz zeigt.

In einem weiteren Ausführungsbeispiel wird die Bestimmung der Glykierung in den Glykierungspositionen der Sequenzen SEQ ID No.1 bis 30 beschrieben. Dabei wird zunächst die Proteinkonzentration in Plasmaproben von Typ-II-Diabetes mellitus Patienten und Nichtdiabetespatienten mittels Bradford-Assay bestimmt. Aliquote entsprechend einer Proteinmenge von 1,2 mg wurden mit 1,5 mL Ammoniumhydrogencarbonat-Puffer (0,1 mol/L, pH 8,0) verdünnt und nachfolgend entsalzt (Vivaspin 2 PES MWCO 5kDa, Sartorius Stedim Biotech). Der Rückstand wurde mit Ammoniumhydrogencarbonat-Puffer (0,1 mol/L, pH 8,0) auf 500 μL verdünnt. 166,7 μL (400 μg) der Lösung wurden mit SDS (0.5% in Wasser, w:v, 20.8 μL) und TCEP (Tris(2-carboxyethyl)phosphinhydrochlorid, 50 mmol/L, 20.8 μL) versetzt und 15 min bei 60°C inkubiert. Die Proben wurden auf Raumtemperatur abgekühlt und mit Iodacetamid (0,1 mol/L, 22.9 μL) unter Lichtausschluss alkyliert (15 min bei Raumtemperatur). Darauf folgt ein enzymatischer Verdau mit Trypsin (25 mg/L in 50 mmol/L Ammoniumhydrogencarbonat-Puffer) bei 37°C. Zunächst werden 800 μL und nach 5 h weitere 320 μL Enzymlösung zugesetzt und für weitere 12h inkubiert. Im Anschluss an den Verdau werden 32 μL der internen Standardlösung (konzentrationsoptimierte Mischung) zugegeben und die Lösung lyophilisiert.

Die Lyophilisate werden in kaltem Puffer (4°C, 50 mmol/L Magnesiumacetat; 250 mmol/L Ammoniumacetat, pH 8,1, 20% (v/v) $CH_3CN$, 100 μL) aufgenommen und auf ein Volumen von 500 μL mit Beladungspuffer (4°C, 50 mmol/L Magnesiumacetat; 250 mmol/L Ammoniumacetat, pH 8,1) verdünnt. Die Proben werden auf eine Polypropylensäule (1 mL; Qiagen) gefüllt mit mAPBA (m-Aminophenyl-boronsäureagarose 1 mL, Säulenbettvolumen) aufgebracht. Nach einem Waschschritt (15 mL Beladungspuffer) wurden die Amadori-Peptide in zwei Schritten mit Essigsäure (0,1 mol/L, 8 mL und 0,2 mol/L, 2 mL) bei 37 °C eluiert und gefriergetrocknet.

Die Lyophilisate werden mit einer wässrigen Acetonitrillösung (0.1% (v/v) Ameisensäure, 60% (v/v), 200 μL) aufgenommen und mit 0.1% (v/v) Ameisensäure sukzessive die Konzentration des Acetonitrilgehalts in Lösung auf 5% (v/v) verringert. Eine Oasis HLB Kartusche (30 mg, 1cc, Waters) wurde mit Methanol (1 mL) und 0,1% (v/v) wässriger Ameisensäure (1 mL) äquilibriert bevor die Probe aufgetragen und die Peptide mit wässrigem Acetonitril (jeweils 333 μL) in Mischungsverhältnissen von 40% (v/v), 60% (v/v) und 80% (v/v) mit einem Zusatz von Ameisensäure (0,1% (v/v)) eluiert werden. Die vereinigten Eluate werden gefriergetrocknet.

Die Glykierungspositionen der Sequenzen SEQ-ID No. 1 bis 30 wird mittels Massenspektrometrie bestimmt. Dazu werden die Lyophilisate mit einer wässrigen Acetonitrillösung (0.1% (v/v) Ameisensäure, 60% (v/v), 10 μL) aufgenommen und mit 0.1% (v/v) wässriger Ameisensäure sukzessive die Konzentration des Acetonitrilgehalts in Lösung auf 5% (v/v) verringert (Endvolumen der Lösung: 120 μL). Aliquote (84 μL) der Lösungen wurden auf eine C18-Säule (AdvanceBio Peptide Map, 150 mm x 2.1 mm, 2.7 μm Partikelgröße; Agilent Technologies, Waldbronn, Germany) aufgetragen und unter Verwendung von einem Waters 2695 Alliance Seperation Module (Waters GmbH, Eschborn, Germany) getrennt (60°C). Eluent A war 0.1% (v/v) wässrige Ameisensäure und Eluent B war 0.1% (v/v) Ameisensäure in CH3CN. Die Säule wurde äquilibriert (3% Eluent B) und die Peptide 3 min nach der Injektion mit einem mehrstufigen linearen Gradienten eluiert: 3 bis 10% Eluent B in 1 min, 10 bis 20% Eluent B in 10 min, 20 bis 95% Eluent B in 8 min. Die Flussrate betrug 300 μL/min. Das Eluat wurde on-line an einem QqLIT-Massenspektrometer (4000 QTRAP LC-MS/MS System, AB Sciex, Darmstadt, Germany) mit einerTurbo V Ionenquelle im Positivionen-Modus analysiert. Die Datenaufnahme beruhte auf einem Mehrfach-Reaktionen-Monitoring (multiple reaction monitoring, MRM) mit drei spezifischen Q1/Q3-m/z-Bereichen für jeden Analyten. Die Analyte wurden über die Integration der Peaks im extrahierten Ionenchromatogramm (extracted ion chromatogramm, XIC) und interner Standardisierung quantifiziert.

Eine Übersicht über die ermittelten Glykierungspositionen ist in der Tab. 1 wiedergegeben. Dabei konnten neben den 5 spezifischen Glykierungspositionen in den Sequenzen SEQ-ID No. 5, 11, 14, 18, 24, 27, 3, 6, 7, 10, 12, 23 und 25 weitere Glykierungspositionen mit signifikant erhöhter Glykierung in den Glykierungspositionen SEQ-ID No 1 bis 30 und 44 bis 46 bei Typ-II-Diabetes mellitus ermittelt werden. Diese Sequenzen könnten zur Stützung der Diagnose bei der Feststellung von Glykierung in zumindest einer Position ausgewählt aus den Sequenzen SEQ-ID No. 5, 11, 14, 18, 24, 27, 3, 6, 7, 10, 12, 23 und 25, bevorzugt zumindest die SEQ ID No. 27 herangezogen werden. Dabei kann die Feststellung der Glykierung in zumindest einer Position ausgewählt aus den Sequenzen SEQ-ID No. 5, 11, 14, 18, 24, 27, 3, 6, 7, 10, 12, 23 und 25, bevorzugt zumindest die SEQ ID No. 27 ein erweitertes Screening zur Feststellung der Glykierung in zumindest einer Sequenz ausgewählt aus den Sequenzen SEQ-ID No. 1 bis 30 und 44 bis 46 nach sich ziehen und eine erfindungsgemäße Absicherung der Diagnostik durch Korrelation mit dem $HbA_{1c}$-Spiegel erfolgen.

**Patentansprüche**

1. Verfahren zur nichtinvasiven Diagnostik von Diabetes, insbesondere Typ-II-Diabetes mellitus, wobei zumindest die Bestimmung der Glykierung in der Glykierungsposition Lys 141 von Humanem Haptoglobin (P00738; SEQ ID No. 38) in einer isolierten Blut- oder Plasmaprobe erfolgt sowie eine Bestimmung des HbA$_{1c}$-Spiegels erfolgt, wobei nachfolgend die Glykierung in den Glykierungspositionen mit dem HbA$_{1c}$-Spiegel korreliert wird.

2. Verfahren nach Anspruch 1, **dadurch gekennzeichnet, dass** die Glykierung von humanen Plasmaproteinen in zumindest einer weiteren Glykierungsposition ausgewählt aus

   - Lys 174 in Humanem Serum Albumin (P02768, SEQ ID No. 31),
   - Lys 414 in Humanem Serum Albumin (P02768, SEQ ID No. 31),
   - Lys 574 in Humanem Serum Albumin (P02768, SEQ ID No. 31),
   - Lys 163 in der Humanen Fibrinogen beta-Kette (P02675, SEQ ID No. 33),
   - Lys 50 in der Humanen Ig Lambda Kette-C-Region (P01842; SEQ ID No. 36),
   in einer isolierten Blut- oder Plasmaprobe bestimmt wird sowie
   - eine Bestimmung des HbA$_{1c}$-Spiegels erfolgt, wobei nachfolgend die Glykierung in den Glykierungspositionen mit dem HbA$_{1c}$-Spiegel korreliert wird.

3. Verfahren nach Anspruch 1 oder 2, umfassend die Schritte:

   - Separation der Plasmaproteine einer Blutprobe,
   - Durchführung eines enzymatischen Verdaus der Plasmaproteine,
   - Bestimmung der Glykierung in einer weiteren Glykierungsposition ausgewählt aus den SEQ ID No. 1 bis 30 und 44 bis 46, bevorzugt ausgewählt aus
   - Lys 174 in Humanem Serum Albumin (P02768, SEQ ID No. 31),
   - Lys 414 in Humanem Serum Albumin (P02768, SEQ ID No. 31),
   - Lys 574 in Humanem Serum Albumin (P02768, SEQ ID No. 31),
   - Lys 163 in der Humanen Fibrinogen beta-Kette (P02675, SEQ ID No. 33),
   - Lys 50 in der Humanen Ig Lambda Kette-C-Region (P01842; SEQ ID No. 36),
   - Lys 93 in Humanem Serum Albumin (P02768, SEQ ID No. 31),
   - Lys 181 in Humanem Serum Albumin (P02768, SEQ ID No. 31),
   - Lys 233 in Humanem Serum Albumin (P02768, SEQ ID No. 31),
   - Lys 378 in Humanem Serum Albumin (P02768, SEQ ID No. 31),
   - Lys 525 in Humanem Serum Albumin (P02768, SEQ ID No. 31),
   - Lys 545 in Humanem Serum Albumin (P02768, SEQ ID No. 31),
   - Lys 120 in Humanen Apolipoprotein A-I (P02647, SEQ ID No. 37),

   oder eines Peptids ausgewählt aus den SEQ ID No. 1 bis 30 und 44 bis 46, bevorzugt, aus den SEQ-ID No. 5, 11, 14, 18, 24, 27, 3, 6, 7, 10, 12, 23 und 25, besonders bevorzugt in der SEQ ID No. 27 sowie Bestimmung des HbA$_{1c}$-Spiegels, wobei nachfolgend die Glykierung in den Glykierungspositionen mit dem HbA$_{1c}$-Spiegel korreliert wird.

4. Verfahren nach einem der vorhergehenden Ansprüche, weiterhin umfassend die Bestimmung des Glykierungszustands mindestens eines weiteren Lysinrests ausgewählt aus den SEQ ID No. 1 bis 30 und 44 bis 46, bevorzugt aus den SEQ ID No. 5, 11, 14, 18, 24, 27, 3, 6, 7, 10, 12, 23 und 25 oder 11, 14, 18 und 24.

5. Verfahren nach Anspruch 1 bis 4, **dadurch gekennzeichnet, dass** nach dem enzymatischen Verdau eine Affinitätschromatographie zur Trennung glykierter Peptide und/oder eine Festphasenextraktion durchgeführt wird.

6. Verfahren nach Anspruch 5, **dadurch gekennzeichnet, dass** als Affinitätschromatographie eine Boronsäurechromatographie durchgeführt wird.

7. Verfahren nach einem der vorhergehenden Ansprüche, **dadurch gekennzeichnet, dass** die Bestimmung der Glykierung in zumindest einer Glykierungsposition mittels Massenspektrometrie, FRET (Förster-Resonanz-Energie-Transfer), ELBIA (Enzym-Linked-Boronate-Immunoassay) oder Immunoassay erfolgt.

8. Verwendung eines glykierten Lysins zur nichtinvasiven Diagnostik von Diabetes, insbesondere Typ-II-Diabetes mellitus, wobei zumindest die Bestimmung der Glykierung in der Glykierungsposition Lys 141 von Humanem Haptoglobin (P00738; SEQ ID No. 38) in einer isolierten Blut- oder Plasmaprobe erfolgt.

9. Kit zur nichtinvasiven Diagnostik von Diabetes, insbesondere Typ-II-Diabetes mellitus, umfassend zumindest ein Reagenz, welches eine Affinität zu zumindest einem Antigen aufweist, welches durch ein Peptid gebildet wird, welches die Glykierungsposition Lys 141 von Humanem Haptoglobin (P00738; SEQ ID No. 38) aufweist, wobei das Antigen im glykierten Zustand detektiert wird.

10. Kit nach Anspruch 9, **dadurch gekennzeichnet, dass** das Reagenz ein Antikörper, Oligonukleotid-Aptamer oder Peptid-Aptamer ist.

11. Kit nach einem der Ansprüche 9 oder 10, weiterhin umfassend zumindest eine immobilisierte Boronsäurekomponente zur Anreicherung von glykierten Proteinen und Peptiden oder einen ELBIA (Enzym-Linked-Boronate-Immunoassay).

12. Verwendung eines glykierten Lysins als Biomarker für die Diagnostik von Diabetes, insbesondere Typ-II-Diabetes mellitus und/oder für die Überwachung einer Therapie von Diabetes, insbesondere Typ-II-Diabetes mellitus, wobei zumindest die Bestimmung der Glykierung in der Glykierungsposition Lys 141 von Humanem Haptoglobin (P00738; SEQ ID No. 38) in einer isolierten Blut- oder Plasmaprobe erfolgt

13. Reagenz aufweisend eine Affinität zu zumindest einem Antigen, welches durch ein Peptid gebildet wird, welches zumindest die Glykierungsposition Lys 141 von Humanem Haptoglobin (P00738; SEQ ID No. 38) sowie flankierende Sequenzabschnitte des entsprechenden Proteins umfasst, wobei das Antigen im glykierten Zustand detektiert wird.

14. Reagenz nach Anspruch 13, **dadurch gekennzeichnet, dass** das Reagenz ein Antikörper, Oligonukleotid-Aptamer oder Peptid-Aptamer ist.


**Claims**

1. Method for the non-invasive diagnosis of diabetes, in particular type II diabetes mellitus, wherein at least the determination of glycation in the glycation position Lys 141 of human haptoglobin (P00738; SEQ ID No. 38) is carried out in an isolated blood or plasma sample and the HbA$_{1c}$ level is determined, wherein subsequently the glycation in the glycation positions is correlated with the HbA$_{1c}$ level.

2. Method according to claim 1, **characterized in that** the glycation of human plasma proteins is determined in at least one further glycation position selected from

   - Lys 174 in human serum albumin (P02768, SEQ ID No. 31),
   - Lys 414 in human serum albumin (P02768, SEQ ID No. 31),
   - Lys 574 in human serum albumin (P02768, SEQ ID No. 31),
   - Lys 163 in the human fibrinogen beta chain (P02675, SEQ ID No. 33),
   - Lys 50 in the human Ig lambda chain C region (P01842, SEQ ID No. 36),
   in an isolated blood or plasma sample and
   - a determination of the HbA$_{1c}$ level is carried out, wherein subsequently glycation in the glycation positions is correlated with the HbA$_{1c}$ level.

3. A method according to claim 1 or 2, comprising the steps:

- separation of the plasma proteins of a blood sample,
- carrying out an enzymatic digestion of the plasma proteins,
- determination of the glycation in one further glycation position selected from SEQ ID No. 1 to 30 and 44 to 46, preferably selected from
- Lys 174 in human serum albumin (P02768, SEQ ID No. 31),
- Lys 414 in human serum albumin (P02768, SEQ ID No. 31),
- Lys 574 in human serum albumin (P02768, SEQ ID No. 31),
- Lys 163 in the human fibrinogen beta chain (P02675, SEQ ID No. 33),
- Lys 50 in the human Ig lambda chain C region (P01842; SEQ ID No. 36),
- Lys 93 in human serum albumin (P02768, SEQ ID No. 31),
- Lys 181 in human serum albumin (P02768, SEQ ID No. 31),
- Lys 233 in human serum albumin (P02768, SEQ ID No. 31),
- Lys 378 in human serum albumin (P02768, SEQ ID No. 31),
- Lys 525 in human serum albumin (P02768, SEQ ID No. 31),
- Lys 545 in human serum albumin (P02768, SEQ ID No. 31),
- Lys 120 in human apolipoprotein A-I (P02647, SEQ ID No. 37),

or a peptide selected from SEQ ID No. 1 to 30 and 44 to 46, preferred, from SEQ ID No. 5, 11, 14, 18, 24, 27, 3, 6, 7, 10, 12, 23 and 25, especially preferred in SEQ ID No. 27, and determination of the $HbA_{1c}$ level, wherein the glycation in the glycation positions is correlated with the $HbA_{1c}$ level.

4. Method according to any of the preceding claims, further comprising the determination of the glycation state of at least one further lysine residue selected from SEQ ID No. 1 to 30 and 44 to 46, preferably from SEQ ID No. 5, 11, 14, 18, 24, 27, 3, 6, 7, 10, 12, 23 and 25 or 11, 14, 18 and 24.

5. Method according to claims 1 to 4, **characterized in that** an affinity chromatography for the separation of glycated peptides and/or a solid phase extraction is carried out after the enzymatic digestion.

6. Method according to claim 5, **characterized in that** a boronic acid chromatography is performed as affinity chromatography.

7. Method according to any of the preceding claims, **characterized in that** the determination of glycation in at least one glycation position is carried out by mass spectrometry, FRET (Forster resonance energy transfer), ELBIA (enzyme linked boronate immunoassay) or immunoassay.

8. Use of a glycated lysine for non-invasive diagnosis of diabetes, in particular type II diabetes mellitus, wherein at least the determination of the glycation at the glycation position Lys 141 of human haptoglobin (P00738; SEQ ID No. 38) is carried out in an isolated blood or plasma sample.

9. Kit for the non-invasive diagnosis of diabetes, in particular type II diabetes mellitus, comprising at least one reagent, comprising an affinity to at least one antigen, which is formed by a peptide comprising the glycation position Lys 141 of human haptoglobin (P00738; SEQ ID No. 38), wherein the antigen is detected in the glycated state.

10. Kit according to claim 9, **characterized in that** the reagent is an antibody, oligonucleotide aptamer or peptide aptamer.

11. Kit according to one of the claims 9 or 10, further comprising at least one immobilized boronic acid component for the enrichment of glycated proteins and peptides or an ELBIA (enzyme-linked boronate immunoassay).

12. Use of a glycated lysine as a biomarker for the diagnosis of diabetes, in particular type II diabetes mellitus, and/or for the monitoring of a therapy of diabetes, in particular type II diabetes mellitus, wherein at least the determination of the glycation in the glycation position Lys 141 of human haptoglobin (P00738; SEQ ID No. 38) is carried out in an isolated blood or plasma sample.

13. Reagent comprising an affinity to at least one antigen, which is formed by a peptide, comprising at least the glycation position Lys 141 of human haptoglobin (P00738; SEQ ID No. 38) and flanking sequence segments of the corresponding protein, wherein the antigen is detected in the glycated state.

**14.** Reagent according to claim 13, **characterized in that** the reagent is an antibody, oligonucleotide aptamer or peptide aptamer.

## Revendications

**1.** Procédé de diagnostic non invasif de diabète, en particulier de diabète sucré de type II, dans lequel au moins la détermination de la glycation en position de glycation Lys 141 de l'haptoglobine humaine (P00738, SEQ ID N° 38) est effectuée dans un échantillon de sang ou de plasma isolé et le niveau d'HbA$_{1c}$ est déterminé, la glycation dans les positions de glycation étant ensuite corrélée au niveau d'HbA$_{1c}$.

**2.** Procédé selon la revendication 1, **caractérisé en ce que** la glycation des protéines plasmatiques humaines est déterminée, dans un échantillon de sang ou de plasma isolé, dans au moins une autre position de glycation choisie parmi

- Lys 174 dans l'albumine sérique humaine (P02768, SEQ ID N° 31),
- Lys 414 dans l'albumine sérique humaine (P02768, SEQ ID N° 31),
- Lys 574 dans l'albumine sérique humaine (P02768, SEQ ID N° 31),
- Lys 163 dans la chaîne bêta du fibrinogène humain (P02675, SEQ ID N° 33),
- Lys 50 dans la région C de la chaîne Ig lambda humaine (P01842, SEQ ID N° 36), et **en ce que**
- le niveau d'HbA$_{1c}$ est déterminé, la glycation dans les positions de glycation étant ensuite corrélée au niveau d'HbA$_{1c}$.

**3.** Procédé selon la revendication 1 ou 2, comprenant les étapes :

- de séparation des protéines plasmatiques d'un échantillon de sang,
- de réalisation d'une digestion enzymatique des protéines plasmatiques,
- de détermination de la glycation dans une autre position de glycation choisie parmi SEQ ID N° 1 à 30 et 44 à 46, de préférence choisie parmi
- Lys 174 dans l'albumine sérique humaine (P02768, SEQ ID N° 31),
- Lys 414 dans l'albumine sérique humaine (P02768, SEQ ID N° 31),
- Lys 574 dans l'albumine sérique humaine (P02768, SEQ ID N° 31),
- Lys 163 dans la chaîne bêta du fibrinogène humain (P02675, SEQ ID N° 33),
- Lys 50 dans la région C de la chaîne Ig lambda humaine (P01842, SEQ ID N° 36),
- Lys 93 dans l'albumine sérique humaine (P02768, SEQ ID N° 31),
- Lys 181 dans l'albumine sérique humaine (P02768, SEQ ID N° 31),
- Lys 233 dans l'albumine sérique humaine (P02768, SEQ ID N° 31),
- Lys 378 dans l'albumine sérique humaine (P02768, SEQ ID N° 31),
- Lys 525 dans l'albumine sérique humaine (P02768, SEQ ID N° 31),
- Lys 545 dans l'albumine sérique humaine (P02768, SEQ ID N° 31),
- Lys 120 dans l'apolipoprotéine humaine A-I (P02647, SEQ ID N° 37),

ou d'un peptide choisi parmi SEQ ID N° 1 à 30 et 44 à 46, de préférence, parmi SEQ ID N° 5, 11, 14, 18, 24, 27, 3, 6, 7, 10, 12, 23 et 25, de manière particulièrement préférée dans SEQ ID N° 27 et le niveau d'HbA$_{1c}$ est déterminé, la glycation dans les positions de glycation étant ensuite corrélée au niveau d'HbA$_{1c}$.

**4.** Procédé selon l'une des revendications précédentes, comprenant en outre la détermination de l'état de glycation d'au moins un autre résidu de lysine choisi parmi SEQ ID N° 1 à 30 et 44 à 46, de préférence parmi SEQ ID N° 5, 11, 14, 18, 24, 27, 3, 6, 7, 10, 12, 23 et 25 ou 11, 14, 18 et 24.

**5.** Procédé selon la revendication 1 à 4, **caractérisé en ce que**, après la digestion enzymatique, une chromatographie d'affinité pour la séparation des peptides glyqués et/ou d'une extraction en phase solide est effectuée.

**6.** Procédé selon la revendication 5, **caractérisé en ce qu'**une chromatographie d'acide boronique est réalisée comme chromatographie d'affinité.

**7.** Procédé selon l'une des revendications précédentes, **caractérisé en ce que** la glycation est déterminée dans au moins une position de glycation par spectrométrie de masse, transfert d'énergie par résonance de type Förster (FRET), immunoessai de boronate enzymatique (ELBIA) ou par immunoessai.

**8.** Utilisation d'une lysine glyquée pour le diagnostic non invasif de diabète, en particulier de diabète sucré de type II, au moins la détermination de la glycation en position de glycation Lys 141 de l'haptoglobine humaine (P00738 ; SEQ ID N° 38) étant effectuée dans un échantillon de sang ou de plasma isolé.

**9.** Kit de diagnostic non invasif du diabète, en particulier du diabète sucré de type II, comprenant au moins un réactif présentant une affinité pour au moins un antigène formé par un peptide ayant la position de glycation Lys 141 de l'haptoglobine humaine (P00738 ; SEQ ID N° 38), l'antigène étant détecté à l'état glyqué.

**10.** Kit selon la revendication 9, **caractérisé en ce que** le réactif est un anticorps, un aptamère oligonucléotidique ou un aptamère peptidique.

**11.** Kit selon l'une des revendications 9 ou 10, comprenant en outre au moins un composant d'acide boronique immobilisé pour l'enrichissement de protéines et de peptides glyqués ou un immunoessai de boronate enzymatique (ELBIA).

**12.** Utilisation d'une lysine glyquée comme biomarqueur pour le diagnostic de diabète, en particulier du diabète sucré de type II et/ou pour le suivi d'une thérapie pour le diabète, en particulier pour le diabète sucré de type II, au moins la détermination de la glycation en position de glycation Lys 141 d'haptoglobine humaine (P00738 ; SEQ ID N° 38) étant effectuée dans un échantillon de sang ou de plasma isolé.

**13.** Réactif présentant une affinité pour au moins un antigène formé par un peptide qui comprend au moins la position de glycation Lys 141 de l'haptoglobine humaine (P00738 ; SEQ ID N° 38) et des sections de séquence flanquantes de la protéine correspondante, l'antigène étant détecté à l'état glyqué.

**14.** Réactif selon la revendication 13, **caractérisé en ce que** le réactif est un anticorps, un aptamère oligonucléotidique ou un aptamère peptidique.

EP 3 077 826 B1

Fig. 1

Fig. 2

Fig. 3

Fig. 4

**HP K141**

Sensitivität

FUK = 77 %
CutOff = 18.9
Sensitivität = 60%
Spezifität = 83%

1 -Spezifität

**HbA$_{1c}$**

Sensitivität

FUK = 86 %
CutOff = 6
Sensitivität = 77%
Spezifität = 94%

1 -Spezifität

Fig. 5

*A*

*B*

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69835268 T2
- EP 0623216 B1
- EP 0230934 A2
- WO 2013159025 A1
- US 20040147033 A1
- WO 2007117794 A2
- US 20110136160 A1